(19) Europäisches Patentamt
European Patent Office
Office européen des brevets

(11) **EP 4 421 158 A1**

(12) **EUROPEAN PATENT APPLICATION**

(43) Date of publication:
**28.08.2024 Bulletin 2024/35**

(21) Application number: **23305244.8**

(22) Date of filing: **23.02.2023**

(51) International Patent Classification (IPC):
*C12M 1/12* (2006.01)  *C12M 1/26* (2006.01)
*C12N 5/00* (2006.01)  *C12N 11/04* (2006.01)
*B29C 64/112* (2017.01)

(52) Cooperative Patent Classification (CPC):
**C12M 25/16; B29C 64/112; B33Y 80/00;**
**C12M 33/00; C12N 5/0075; C12N 5/0667;**
**C12N 11/04;** C12M 27/02; C12N 2533/54;
C12N 2533/74; C12N 2533/80; C12N 2537/10

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **SARTORIUS STEDIM FMT SAS**
**13400 Aubagne (FR)**
• **UNIVERSITE CLAUDE BERNARD - LYON 1**
**69100 Villeurbanne (FR)**
• **Institut National des Sciences Appliquées de Lyon**
**69621 Villeurbanne Cedex (FR)**

• **Ecole Superieure de Chimie Physique Electronique de Lyon**
**69616 Villeurbanne, Cedex (FR)**
• **Centre National de la Recherche Scientifique**
**75016 Paris (FR)**

(72) Inventors:
• **Petiot, Emma**
**69100 Villeurbanne (FR)**
• **Chocarro**
**69008 Lyon (FR)**
• **Courtial, Edwin-Joffrey**
**69100 Villeubanne (FR)**

(74) Representative: **Mewburn Ellis LLP**
**Aurora Building**
**Counterslip**
**Bristol BS1 6BX (GB)**

(54) **MICROCARRIERS FOR CELL CULTURE**

(57) Methods for producing microcarriers by ink jetting, comprising propelling a droplet of a solution comprising one or more biomaterials capable of forming a hydrogel and one or more cells, into a liquid medium. Microcarriers produced by such methods, methods in which such microcarriers are used to culture a cell population in a bioreactor and a system for producing microcarriers by ink jetting are also disclosed.

Fig. 5

EP 4 421 158 A1

**Description**

<u>Field of the Present Disclosure</u>

The present disclosure relates to cellularized microcarriers, methods for production of such microcarriers, and methods for the industrial production of cells using such microcarriers, wherein the cells are included in a hydrogel matrix. Related methods, systems and products are described.

<u>Background</u>

Bioprocesses use biological systems to produce specific bio-products with therapeutic effects, such as cells or biomolecules. This process typically involves placing cells and/or microbes into a bioreactor with media containing nutrients under controlled atmospheric conditions. In the context of bioproduction, bioprocesses are typically performed as large-scale culture processes in stirred tank bioreactors comprising liquid medium, in which the cells are in suspension or cultured on the surfaces of microcarriers. Stirred tank bioreactor processes impose some physical limitations on the cellular density that can be achieved, and hence ultimately the productivity of the system. Indeed, in stirred cultures (whether with cells in suspension or on microcarriers), the supply of gasses and nutrients to the cells becomes limiting as the cell density increases, as the supply and agitation means can damage the cells if operated at high rates (e.g. due to the shear stress caused by the moving medium and/or rotating shaft). Thus, there is a trade-off between the density of cells that can be effectively sustained, and the physical damage caused by the systems that provide the gasses and nutrients to sustain such densities. This problem is often mitigated by introducing compounds that protect the cells from hydrodynamic shear stress (such as e.g. polymeric surfactants), which may cause problematic in downstream processing.
In many static culture systems, unlike microcarriers which are suspended in nutritive liquid, the surface area available for cell growth is limited by surface of adhesion. On the contrary the use of microcarriers increases the surface area available for growth, particularly if cells are able to grow both on the external and internal surface of the microcarrier. In existing microcarrier systems, cells experience shear stress and shocks during culture, and proliferation capacity is often limited to certain surfaces of the microcarrier. In some microcarrier systems, additional fresh microcarriers are added to provide further space for cell expansion, however this increases the frequency of bead-to-bead shocks, exacerbating the stress that cells experience. Cell recovery from microcarrier systems is also limiting, and the processes used, such as trypsinisation, can damage cells and lead to reduced viability in the harvested cell product. Additionally, existing systems often require multiple steps throughout the culture process, including production of the microcarriers, initial seeding of the microcarriers with cells, followed by cell expansion onto the microcarriers, and finally a cell recovery process. All of these steps are time-consuming, and any manual intervention required during the culture process increases the opportunity for contamination, and/or a reduction in the purity of the final product. These issues are particularly problematic when amplifying a fragile cell population, such as stem cells. Stem cells are particularly susceptible to shear stress and shock, which can induce undesirable differentiation of the population.
Therefore, a need exists for an improved microcarrier system that addresses one or more of the above problems.

<u>Summary</u>

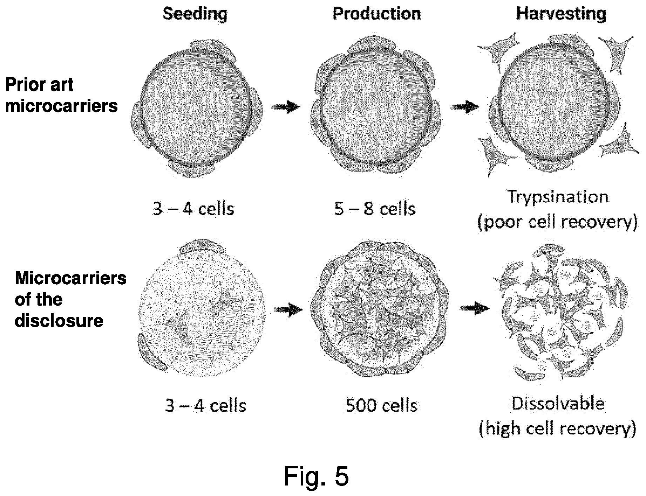

The present disclosure provides a customisable microcarrier system that integrates the benefits of increased proliferation capacity, cell-protective properties, and improved cell recovery (illustrated on Figure 5). The present inventors identified that the production of cellular products inside microcarriers obtained by forming a bioink droplet through inkjet bioprinting could solve many problems faced by traditional microcarrier systems, enabling the effective amplification of even fragile cell types such as stem cells in suspension bioreactor culture systems. The present inventors have previously developed a bioink formulation for 3D printing of tissues, in particular skin substitutes (US 2019/0002836; Pourchet et al., Adv. Healthcare Mater. 2017, 1601101). The present inventors have further demonstrated that this formulation could be used to 3D print structures that can be used as support for high scale growth of production cells (PCT/EP2022/073485). The inventors hypothesised that the bioink formulation could be used to make microcarriers for use in stirred tank bioreactors, where cells are cultured in suspension. The inventors utilised this bioink formulation to form microbeads loaded with cells using an inkjet system to dispense droplets of the bioink directly into a liquid medium, producing bead-like hydrogel structures on a micrometre scale. The inventors showed that they were able to produce microbeads with cells distributed throughout the hydrogel structure, *i.e.* microbeads with cells attached to the external surface as well as embedded within the body of the hydrogel structure. Such microbeads were shown to enable a 3- to 4-fold increase in cell numbers over 21 days of culture. As the cells proliferated, the inventors observed that cells were able to proliferate throughout the beads, for example forming "web-like structures" within the beads and populating the bead surface. Additionally, certain cell types, such as fibroblasts, were able to displace and remodel the hydrogel matrix, by secreting extracellular factors such as metalloproteases that break down the gelatin and alginate polymers. This is advantageous and demonstrates that the hydrogel microcarriers used according to the disclosure can maximise the space available for cells to colonise, by (i) enabling homogenous seeding of the cells throughout the volume of the microbead structure, (ii) enabling the cells to grow in 3 dimensions within the beads as well as on the surface of the beads, and in some cases (iii) enabling the colonisable space to increase during culture, as the hydrogel structure of the microbead is remodelled. The inventors also demonstrated that their microbead culture system was particularly advantageous for the culture of stem cells, as embedding cells within the volume of the microbead structure protected them from bead-to-bead shocks experienced by cells adhered to the external surface of a microcarrier, and also shear stresses caused by eddy currents within stirred tank bioreactor systems. The inventors also recognised that the approach could enables efficient recovery of cells embedded in the structure thanks to enzymatic degradation of the outer hydrogel with limited cell viability loss. Having realised the advantages associated with using microbeads produced by ink-jetting a hydrogel bioink, the inventors designed a system wherein microbeads were ink jetted directly into the bioreactor, or into a collection bath that directly feeds the bioreactor. This is advantageous as an enclosed system requiring less manual intervention reduces the likelihood of contamination, ensuring a high purity cell product.

In a first aspect of the disclosure, there is provided a method of producing a microcarrier for cell culture, the method comprising: providing a solution comprising one or more biomaterials capable of forming a hydrogel and one or more cells; and propelling a droplet of the solution into a liquid medium by ink jetting, thereby producing a cellularised microcarrier.

The heterogeneity of stem cell types as well as the numerous areas of application (such as allogenic or autologous therapies) suggest that different production processes will likely be necessary for their large-scale culture *in vitro.* The present methods advantageously enable such flexibility as parameters such as the size, composition and cellular density of the droplet can all be adjusted to provide the most suitable environment for any particular cell population. For example, the size of the droplet can be adapted by changing the size of the printing nozzle, and/or one or more printing parameters (e.g. timing of opening of a valve) and/or the viscosity of the solution (e.g. by increasing the amount of one or more biomaterial capable of forming a hydrogel, such as e.g. gelatin, as will be explained further below, and/or by changing the temperature during printing). Further, the solution can include one or more compounds that improve the viability and/or maintain stemness of cells. Indeed, the solution can be prepared using culture medium (as will be explained further below), which can include factors to maintain stemness of the cells. Further, the droplets can be consolidated to a smaller or larger extent, by influencing the amount of cross-linking in the hydrogel matrix. This in turn may impact the stiffness of the microcarrier, offering more protection for shocks and/or a stiffer attachment surface. Conversely, a less cross-linked hydrogel matrix may enable increased proliferation of the cells.

The method of the first aspect may have any one or any combination of the following optional features.

Providing the solution may comprise mixing a liquid cell culture medium, one or more cells, and at least one solution comprising a biomaterial capable of forming a hydrogel. Providing the solution may further comprise incubating the solution at a predetermined temperature for a predetermined period of time prior to ink jetting (or, equivalently, bringing the solution to a predetermined temperature), and/or maintaining the solution at a predetermined temperature during jetting, such that the rheological properties of the bioink composition are compatible with printing without loss of cell viability. The solution may be incubated at and/or brought to a temperature between 30°C and 40°C prior to printing. The solution may be maintained at a temperature between 20°C and 40°C during printing. Providing the solution may comprise providing a solution comprising a liquid culture medium and one or more cells. The one or more cells may have been mechanically and/or enzymatically separated. The present inventors have found that separation of the cells prior to including the cells in the solution for printing reduced the risk of clogging of the printing system. The solution comprising one or more biomaterials capable of forming a hydrogel and the one or more cells (optionally themselves provided in solution) may be mixed to form a homogenous solution (a "bioink", or "bioink solution"). Incubating the solution for a predetermined period of time at a temperature between 30C and 40C prior to printing may further ensure the homogeneity of the solution. This may also enable the cells to recuperate after dissociation and mixing and prior to printing. A homogeneous solution is advantageous as cells are equally distributed throughout the droplet when it is formed, thereby improving the growth potential and reducing the risk of undesirable effects due to cell crowding (such as e.g. differentiation of pluripotent cells, stress, etc.). Obtaining a homogeneous solution also ensures that similar numbers of cells are embedded within each droplet formed, leading to homogenous growth conditions amongst the microcarriers. This enables for example optimisation of the harvesting protocol, as all microcarriers will reach similar cell densities at approximately the same time. This is particularly advantageous when dealing with stem cells, which can differentiate when they receive signals induced by cell contact. The viscosity of the solution may be optimised such that the rate of cell sedimentation during the printing process is negligible.

A cellularised microcarrier produced according to the present aspect may comprise a hydrogel matrix and one or more cells at least partially embedded within the hydrogel matrix. The cellularised microcarrier may comprise cells embedded throughout the entire volume of the microcarrier. A majority of the cells may be completely embedded within the hydrogel matrix when the microcarrier is formed. During proliferation, the cells may divide and migrate to colonise different regions of the microcarrier, advantageously maximising the available space for proliferation. Any cell of the one or more cells may be entirely embedded within the hydrogel matrix, partially embedded within the hydrogel matrix, or may adhere to the external surface of the hydrogel matrix. Due to their formation from a droplet of bioink, the cellularised microcarriers do not comprise a hollow centre, mechanically formed pores (although micropores may be formed due to the action of the cells and/or dissolution of hydrogel components during culture), or an internal surface for cell adhesion. The entire volume of the microcarrier may comprise a hydrogel matrix. This is advantageous as distributing the cells throughout the entire volume of the microcarrier greatly increases the total cell capacity that can be reached, compared to microcarriers wherein cells are grown adhered to an internal or external surface. This in turns means that the microcarrier density that may be needed to achieve a particular overall cell density may be lower than with conventional microcarriers. As a consequence, the cells experience less shocks and shear stress during culture, improving cell viability and reducing the risk of differentiation of pluripotent cells. Additionally, such a microcarrier provides homogeneous growth conditions for all cells, and is simple to manufacture at scale. Furthermore, cells that are entirely embedded within the hydrogel matrix are advantageously protected from shear stress and shocks caused by microcarrier collisions.

Use of inkjet printing enables easy sterilisation (as the flow path of an inkjet printer, such as e.g. cartridge and nozzle can be sterilised or single use), and high throughput production of droplets while maintaining high cell viability. For example, an inkjet printer typically comprises channels that are wider than those of microfluidic systems, with a single narrow point at the nozzle of the printer (compared to micrometric channels through the fluid paths of a microfluidic device), which is advantageous as shear stress during the printing process is minimal, as shown herein.

The one or more cells may comprise or consist of one or more stem cells. The stem cells may be adherent stem cells. The stem cells may be susceptible to mechanically induced differentiation, and/or differentiation induced by cell contact. The stem cells may be an immortalised cell line. Advantageously, the stem cells may be primary cells. The stem cells may be isolated from a sample previously obtained from a patient and thereafter grown or otherwise processed. Processing the stem cells may comprise trypsinisation to separate the cells, and/or washing the cells, and/or an isolation step to obtain a pure stem cell population. Any cell processing steps that are used prior to conventional adherent culture of stem cells may be used in combination with the present methods. For example, an isolation step may be performed by known methods, e.g. FACS. Culturing stem cells in accordance with the first aspect is advantageous as there is no gold standard method for their large-scale production, e.g. for therapeutic applications. While there are many solutions for culturing other cell types in industrial bioprocesses, these are not suitable for stem cells because of their sensitivity to stress, which can induce differentiation. The present inventors have shown that the methods described herein enable the culture of stem cells with high viability and maintaining pluripotency.

The one or more cells may be present at a concentration of at least 0.5 million cells / mL, at least 1 million cells / mL, or between 1 and 3 million cells /mL. The present inventors have found that these cell seeding densities enabled printing with high viability and homogeneity, with high growth potential in culture (i.e. leading to high titre of cells at the end of the culture).

The solution may further comprise a liquid culture medium. The solution may have been obtained by mixing one or more solutions each comprising biomaterials capable of forming a hydrogel and/or cells in a liquid culture medium. The liquid culture medium may be a stem cell culture medium. A stem cell culture medium may be a medium that has been formulated to maintain pluripotency. Such a medium may comprise one or more compounds or compositions that maintain stemness. The medium may comprise factors that promote cell proliferation, for example, FBS, platelet lysate, non-essential amino acids (NEAAs), FGF2, Activin, IGF, MEF-CM, LIF, BMP4, PDGF, EGF, and/or TGF-β. The medium may be a commercially available stem cell culture medium, such as DMEM, MSC GROWTH 2, or MSC NutriStem® XF. The commercially available stem cell culture medium may comprise one or more factors that promote cell proliferation.

The droplet may have a diameter between 100μm and 2mm, between 100μm and 1.5mm, between 100μm and 1mm. The droplet may have a diameter between 100μm and 800μm. The droplets may comprise a volume of solution below 5 μl, below 1.7 μl, below 0.5 μl, or between 0.5 nl and 0.3 μl. The droplet may have a maximum diameter of 2mm. The droplet may be spherical or non-spherical. The droplets may comprise a volume of solution between 0.5 nl and 5 μl, between 0.5 nl and 1.7 μl, between 0.5 nl and 0.5 μl, or between 0.5 nl and 0.3 μl. The droplets may comprise a volume of solution between 0.5 nl and 0.5 μl. The droplets may comprise a volume of solution between 0.5 nl and 0.32 μl. The droplets may have a volume below 5 μl, below 2 μl, below 1.5 μl, below 1 μl, or below 0.5 μl. The diameter of a droplet refers to the diameter of the smallest sphere that the droplet fits into. When the droplet is spherical, this may be the true geometric diameter of the droplet. The diameter of a population of droplets refers to the average diameter of the droplets in the population. Reference to a droplet having a particular diameter refers to the droplets formed through the method having an average diameter equal to the particular diameter. The size of the droplets depends at least in part on the size of the ink jetting nozzle. Wider nozzles result in larger droplets and reduce the shear stress experienced by the cells during printing. However, larger droplets may limit the diffusion of nutrients and gasses to the cells during cell culture. The present inventors have found the above ranges of sizes to be particularly beneficial in striking a balance between these factors.

The method may further comprise consolidating the droplet. Consolidating the droplet may comprise exposing the droplet to one or more solutions that cross-link one or more of the biomaterials capable of forming a hydrogel.

Consolidating may produce a microstructure comprising a hydrogel matrix and the one or more cells. The liquid medium in which the droplet is propelled may comprise one or more compounds capable of cross-linking one or more of the biomaterials capable of forming a hydrogel. The compounds that are capable of cross-linking one or more of the biomaterials may depend on the particular biomaterial used. For example, when the one or more biomaterials comprise alginate, the compounds may comprise a calcium salt. As another example, when the one or more biomaterials comprise gelatin, the compounds may comprise transglutaminase. As another example, when the solution comprises fibrinogen as an additive, the solution for cross-linking may further comprise thrombin. A separate consolidation step is optional and depends on the biomaterials used. For example, when using a solution comprising alginate and gelatin, consolidation is advantageous as gelatin does not form a stable hydrogel at temperatures suitable for mammalian cell culture. By contrast, alginate once consolidated by the presence of calcium, forms a hydrogel that remains stable in such conditions. Therefore, gelatin may be included in the solution to maintain the structure of the droplet while it is being formed, and the droplet may be consolidated by exposure to calcium ions, for example included directly in the liquid medium in which the droplet is jetted.

The step of propelling a droplet of the composition may be performed at a temperature between 20 and 40C. The step of propelling a droplet of the composition may be performed at a temperature between 30 and 40C. The temperature may be below 40C. Temperatures above 40C may be detrimental to cell viability and/or negatively affect the rheology of the solution. For example, when the solution comprises gelatin, temperatures above 40C may be above the glass transition temperature of the gelatin, thereby preventing the gelatin from maintaining the solution at a printable viscosity. The temperature may be set such that the solution shows Newtonian behaviour. Viscous behaviour of a solution can be assessed using a rheometer as known in the art and as described herein. The solution may also be kept at a temperature within these ranges while the composition is prepared for jetting, immediately before the step of propelling a droplet of the composition. The liquid medium in which the droplet is jetted may be at a temperature between 20°C and 40°C. The choice of a temperature within this range to be used during printing may depend on the specific composition of the solution. For example, solutions that are more viscous may benefit from higher temperatures within that range to enable jetting and control shear stress. For example, solutions comprising up to 5% w/v gelatin may be maintained at temperatures between 30 and 40C during printing, whereas solutions comprising higher % gelatin (such as e.g. 10% w/v gelatin) may benefit from temperatures between 35°C and 40°C.

The method may further comprise maintaining the liquid medium comprising the droplet at a temperature between 30°C and 40°C for a predetermined period of time. This may ensure good consolidation of the droplet. The method may comprise washing the cellularised microcarrier (e.g. after consolidation), for example using a buffer (e.g. NaCl 0.9%). The method may further comprise separating the cellularised microcarrier from the liquid medium or the washing medium, and resuspending it in growth medium.

The droplet may be propelled using an inkjet printer with a fluid pressure between 0.25 and 5 bar, between 2 and 5 bar, optionally between 3 and 4 bar. The present inventors have found that a printing pressure between 0.25 and 5 bar is able to balance the requirements of achieving a high jetting rate of the composition, while minimising shear stress the cells experience during the jetting process. The jetting conditions may be adjusted based on the particular formulation of the solution for printing, and its rheological properties. For example, a more viscous solution may benefit from jetting at a higher temperature and/or pressure, while a less viscous solution may be jetted at a lower temperature and/or pressure. The jetting conditions may be adjusted to produce microcarriers of different size.

The droplet may be propelled using an ink jetting device with a valve opening time between 5 and 50ms, such as e.g. about 10 ms.Such valve opening frequencies were found to be suitable for the consistent formation of well separated droplets of bioink compositions as described herein. The droplet may be propelled using a flow rate between about 9μL/s and about 20μL/s, or between 15 μL/s and 20μL/s. These flow rates were found to be compatible with jetting of bioink to form consistent droplets at a pressure compatible with cell viability (e.g. up to 4 bar). For example, at printing temperatures between 30°C and 37°C, solutions comprising between 2.5%w/v gelatin and 5%w/v gelatin were printable at a flow of at least 18μL/s at 4 bar. Solutions comprising less than e.g. 3%w/v gelatin may be printable at lower pressures e.g. 3, 2 or even 1 bar, with such flow rates.

The biomaterial capable of forming a hydrogel may comprise alginate and/or gelatin. The solution may comprise alginate and gelatin. The solution may comprise between 2% and 10% w/v of gelatin, and between 0.5% and 3% w/v of alginate. The solution may comprise about 5% w/v gelatin and about 2%w/v alginate, or about 2.5% w/v gelatin and about 2% w/v alginate. Other biomaterials capable of cross-linking to form a hydrogel are possible and envisaged. For example, collagen gels may be used. The use of alginate and/or gelatin is advantageous because the gelling behaviour can be easily controlled and is stable in cell culture. By contrast, the structure of collagen gels is pH dependent and collagen gels when exposed to 37C temperatures, As such, use of collagen requires specific conditions during printing, such as control of pH to prevent gelling. The presence of gelatin enables the composition to have the desired viscosity to be able to form a droplet that maintains its shape until the droplet is consolidated. The gelatin itself and/or the alginate can be consolidated, for example by exposing the droplets to transglutaminase and/or a solution comprising a calcium salt, which respectively consolidate the gelatin and the alginate. The present inventors have found that the above concentrations are particularly advantageous as higher concentrations can lead to a composition that is too viscous to be printed while maintaining cell viability, whereas lower concentrations may not be amenable to forming droplets and/or may lead to droplets that do not have enough structural integrity.

The hydrogel when formed (i.e. either directly or after consolidation) may be dissolvable using one or more enzymatic solution(s). The enzymatic solution(s) may comprise one or more of collagenase (e.g. collagenase A), alginate lyase, papain and dispase. The solution may comprise collagenase at a concentration between 0.5% and 3% w/v. Concentrations of collagenase below 3% are advantageous as higher concentrations may damage the cells. When the one or more enzymatic solution(s) comprise dispase, the concentration of dispase may be between 0.2-2 U/ml, such as e.g. 0.8 U/ml. The enzymatic solution(s) may not include trypsin. The method according to the present aspect may comprise a step of partially dissolving the hydrogel before or during culture of the cells. For example, the hydrogel may be partially dissolved by dissolving the gelatin, such as by exposing the consolidated droplets to temperatures above the melting temperature of the gelatin (e.g. when the gelatin has not been cross-linked, and where this is compatible with cell viability). The gelatin may be enzymatically dissolved using collagenase, dispase and/or papain. Alginate may be dissolved using alginate lyase. Collagen may be dissolved using collagenase.

The viscosity of the solution comprising one or more biomaterials capable of forming a hydrogel and one or more cells may be below 5 Pa.s, below 1 Pa.s or between 0.05 and 0.7 Pa.s. The present inventors have found that solutions with a viscosity within the range of 0.05 and 0.7 Pa.s are best suited to the ink jetting process, as this viscosity enables jetting of the solution at a high flow rate, while minimising the shear stress cells experience. The viscosity of the solution may be adjusted by adjusting the temperature and/or pressure during the jetting process. The viscosities provided may refer to the viscosity during printing.

The solution may comprise at most 5% w/v gelatin. The solution may comprise at most 3% w/v of gelatin. The present inventors have identified that compositions with such low percentages of gelatin were printable, leading to well-formed cellularised microcarriers while having low enough viscosity that shear stress during printing was low enough to maintain high cell viability and stemness in the case of stem cells. Shear stress can be measured using a rheometer, or by computational modelling. The printing parameters and/or the composition of the solution for printing may be optimised to maintain the shear stress at a level below $5 \cdot 10^3$ Pa, such as between $9.71 \cdot 10^{-1}$ and $4.87 \cdot 10^3$ Pa. Such levels of shear stress are believed to not negatively affect cell viability. The solution may have a Newtonian fluid behaviour, which advantageously means that the viscosity of the solution does not change during the printing process (for example, when the solution passes through the small aperture of the nozzle).

The solution may further comprise a compound or composition that promotes cell adhesion. The compound or composition may be selected from fibrinogen, Dermial®, hyaluronic acid, chitosan, collagen I, and combinations thereof. When fibrinogen is present in the composition, the method may further comprise exposing the droplet to a solution comprising thrombin. Thombin advantageously promotes polymerisation of fibrinogen, which can be utilised during consolidation of the microcarriers.

The method may comprise collecting the droplet in a non-static liquid medium. The liquid medium may be in an agitated vessel such as a bioreactor. The liquid medium into which the droplet is propelled may be agitated. Agitation of a liquid medium may be performed with an impeller. The present inventors have identified that it was beneficial to jet the droplets into a non-static bath to prevent the droplets from aggregating. They have further found that an agitated vessel where a small vortex can be created, such as e.g. in a bioreactor for cell culture in suspension, was an advantageous set up as this enabled to prevent aggregation and to further culture the cells. The vessel may be located below the jetting location at a predetermined distance from the ink jetting location (e.g. location of the nozzle of an ink jetting device), such that droplets fall a distance before entering the liquid collection medium. The distance may be at least 4 cm. The depth and volume of the liquid may be adjusted depending on the amount and size of droplets to be jetted. The liquid may have a minimum depth of 2 cm, to prevent distortion and/or flattening of the droplets as they are propelled into the liquid (e.g. avoiding contact between the droplets and the bottom of the vessel containing the liquid medium), and build-up of the droplets at the bottom of the vessel comprising the liquid medium. The vessel may be maintained at a temperature to optimize cell viability, and maintenance of the structural integrity of the newly printed droplets. The liquid medium may be maintained at a temperature between 20 and 37 °C. The liquid medium may be maintained at 37 °C at the start of the printing process, and subsequently be left to equilibrate to room temperature during jetting.

The solution may comprise about 2% w/v fibrinogen, about 2.5% w/v alginate, and about 2.5% w/v gelatin. In such embodiments, the step of propelling a droplet of the composition may be performed at a temperature between 30°C and 40°C and/or in conditions such that the viscosity of the solution is between 0.06 - 0.12 Pa.s. The solution may comprise about 2 % w/v fibrinogen, about 2% w/v alginate, and about 5% w/v gelatin. In such embodiments, the step of propelling a droplet of the composition may be performed at a temperature between 30C and 40C and/or in conditions such that the viscosity of the solution is between 0.15 and 0.63 Pa.s. The solution may comprise about 2% w/v fibrinogen, about 1% w/v alginate, and about 10% w/v gelatin. In such embodiments, the step of propelling a droplet of the composition may be performed at a temperature between 35°C and 40°C and/or in conditions such that the viscosity of the solution is between 0.11 - 0.63 Pa.s.

The solution may comprise one or more additives selected from: Ginsenoside Rg1, L-proline, a surfactant (for example Pluronic), one or more growth factors (for example FGF-2; human platelet lysate), vitamins (for example ascorbic acid), and fibrous biomaterials (for example, nanocellulose). Pluronic may be added to reduce shear stress and foaming in stirred suspension cultures, such as a stirred tank bioreactor. Ascorbic acid may be added to promote both proliferation and maintenance of the expression of stem cell markers. Ascorbic acid may reduce differentiation of the stem cells during culture. Ginsenoside Rg1 and/or L-proline may be added to promote proliferation and prevent and/or modulate differentiation. Fibrous biomaterials may be added to promote cell adhesion to the microcarrier. The bioink solutions according to the present aspect may comprise any one or more of the additives, in combination with any one or more additional growth factors described herein.

Propelling a droplet of the composition may be performed using a continuous ink jetting device. Propelling a droplet of the composition may be performed using a device comprising a piezoelectric valve. Propelling a droplet of the composition may be performed in a sterile environment (e.g. at least using sterilised equipment and/or a sterilised flowpath). Propelling a droplet of the composition may be performed using an apparatus configured such that every element of the apparatus flow path is sterilisable. The pressure microinjector may comprise a piezoelectric actuation system, for forming and dispensing droplets of the solution. The piezoelectric actuation system may be a Pico Pµlse system. The inkjet printer may comprise a cartridge for receiving the solution for printing. The Pico Pµlse system is advantageously modular, such that each component of the flow path can be easily sterilised or single use. Propelling a droplet may comprise a continuous purge of the solution through a nozzle. Alternatively, the nozzle may comprise a valve, and propelling a droplet may comprise applying pressure to the solution whilst opening and closing the valve. A continuous purge refers to a continuous flow of the solution through the inkjet device, wherein droplets are formed by the surface tension of the solution and shear force at the nozzle. Preferably, the nozzle comprises a valve to direct the formation of droplets by the controlled opening and closing of the valve. Using a valve advantageously improves the size uniformity of the droplets and prevents aggregation of the droplets during jetting.

Propelling a droplet may comprise applying pressure on the solution passing through a nozzle. The nozzle may have a diameter between about 50µm and about 300 µm, between about 50µm and about 200 µm, or between about 50µm and about 100 µm. The nozzle may have a diameter of about 50 µm and the droplet may have a diameter between 100 µm and 400 µm. The nozzle may have a diameter of about 100 µm and the droplet may have a diameter between 400 µm and 1000 µm. Thus, the size of the nozzle used may influence the size of the droplet formed. The nozzle may be made of ceramic, plastic or metal, such that it can be easily sterilised.

According to a second aspect of the disclosure, there is provided a cellularised microcarrier obtained or obtainable through the method of any embodiment of the first aspect.

According to a third aspect of the disclosure, there is provided a method for performing a bioproduction process comprising the production of a cell population, wherein the process comprises maintaining a microcarrier according to the second aspect in suspension in a culture medium in a culture vessel. The method may comprise producing the microcarrier according to any embodiment of the first aspect. Thus, the method may comprise performing the method of any embodiment of the first aspect to obtain the microcarrier.

The culture vessel may be an agitated bioreactor. The method may further comprise culturing the cells for at least 3, 5, 7, 14, 21, or 28 days, or about 14, days, 21 days, 28 days, 35 days, 42 days, 49 days, 60 days, about 1 month or about 2 months The cells may be stem cells and culturing the cells may comprise maintaining the cells in an undifferentiated state. The culture vessel may be an agitated culture vessel. The culture vessel may be a bioreactor. The method may comprises maintaining the cells for at least 4, at least 10 or at least 20 amplifications. Maintaining the cells may comprise maintaining the cells at least partially embedded in the microcarrier in suspension in the culture medium without adding additional physical support for cell growth. The cells may undergo a plurality of amplifications and thereby colonise the microcarrier. Thus, the cells may be maintained until the cell population is at least 4 times, at least 10 times, at least 20 times or at least 30 times the size of the cell population present in the microcarriers at the start of the culture process. The present methods may enable the obtaining of high numbers of amplifications without addition of further physical support for the cells to grow on. This is by contrast to conventional microcarriers where the cells grow on external surfaces (including pore surfaces), where achieving high numbers of amplifications require the addition of fresh microcarrier. This is advantageous because the addition of fresh microcarrier increases the shocks experienced by the cells due to an increasing density of microcarriers. This can negatively impact viability and/or stemness, as some stem cells differentiate in stress conditions such as when experiencing shocks. By contrast, according to the methods of the disclosure, the cells are able to grow in an undifferentiated state within the microcarrier, gradually colonising the hydrogel matrix and replacing its volume by consuming the biomaterials therein. Thus, the present system enables the obtaining of high densities of cells per microcarrier particle, with good stemness and viability.

The cells may be primary cells. The cells used to produce the microcarrier may have been collected at any passage from their isolation from patients up to their optimal growth passages in cell culture. In embodiments, the cells used to produce the microcarrier may be stem cells and/or primary cells that have been cultured for 1 to 6 passages prior to inclusion in the microcarrier. Advantageously, stem cells collected between passages 4 and 6 were demonstrated to have the maximum potential for viability and expansion without differentiation.

As the cells undergo amplifications, they may gradually replace the volume of the hydrogel matrix present in the microcarrier. The cells may remodel the hydrogel matrix, and/or break down the polymers forming the matrix, thus dissolving it. This is advantageous as the cells displacing and/or dissolving the microcarriers provides the cells with more room for expansion and cell proliferation, without requiring the addition of fresh microcarriers. The cells may secrete extracellular matrix components. The surface of the microcarrier may become coated in extracellular matrix. This is advantageous as it protects the cells near the outer edge of the microcarrier from shear and shock, even once the hydrogel matrix is dissolved.

The method may further comprise recovering the cell population by at least partially dissolving the hydrogel matrix using one or more enzymatic solution(s). The enzymatic solution(s) may comprise one or more of: collagenase (e.g. collagenase A), alginate lyase, papain and dispase. The method may or may not comprise recovering the cell population with trypsin. When the cell population is harvested before reaching confluency, trypsin may not be required to separate cells, due to minimal cell-cell contacts. The cell product may therefore be recovered using only enzymatic dissolution of the hydrogel matrix. The recovery of cells by dissolving the hydrogel matrix is a gentle recovery process that improves cell viability, which is crucial for stem cells that are typically used as a product themselves. By contrast, cell recovery from conventional microcarriers typically involves trypsinisation, which is known to damage cells. Alternatively, trypsin may be used in some embodiments to break up the cell mass produced by the culture end point. When a plurality of enzymes is used, such as e.g. collagenase and alginate lyase, it is advantageous for the microcarriers to be incubated with both enzymes at the same time. This may reduce the total incubation time, thereby reducing the stress to the cells. The step of at least partially dissolving the hydrogel matrix may be performed in the culture medium in which the cells are cultured. This may be performed directly in the culture vessel. Alternatively, the microcarrier particles may be recovered through a physical process (such as e.g. centrifugation, filtration), optionally washed, then the hydrogel matrix can be dissolved using one or more enzymatic solutions.

According to a fourth aspect of the disclosure, there is provided a system comprising: a bioreactor; and an inkjet printing device coupled with the bioreactor or with a microcarrier recovery tank fluidically connected with the bioreactor, wherein the inkjet printing device, bioreactor and optionally microcarrier recovery system are all connected in a single sterile system and wherein the inkjet printing device is configured to dispense droplets of liquid directly into a liquid medium in the bioreactor or in the microcarrier recovery tank. The bioreactor may be a stirred tank. The system may further comprise a separation system, such as e.g. a filter or centrifuge, fluidically connected between the microcarrier recovery tank and the bioreactor. The recovery tank may comprise a washing system, such as e.g. a tank with a flow of washing solution, optionally coupled with a separation system.

Brief Description of the Drawings

Embodiments of the present disclosure will now be described by way of example with reference to the accompanying drawings in which:

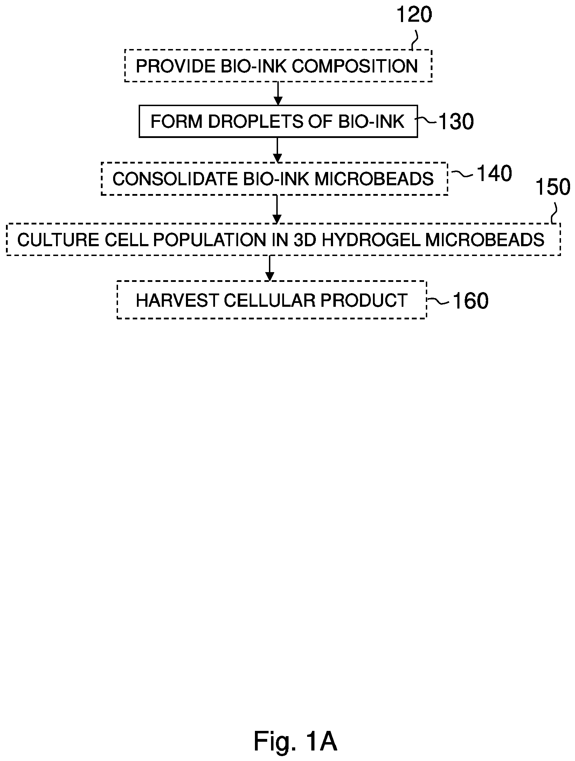

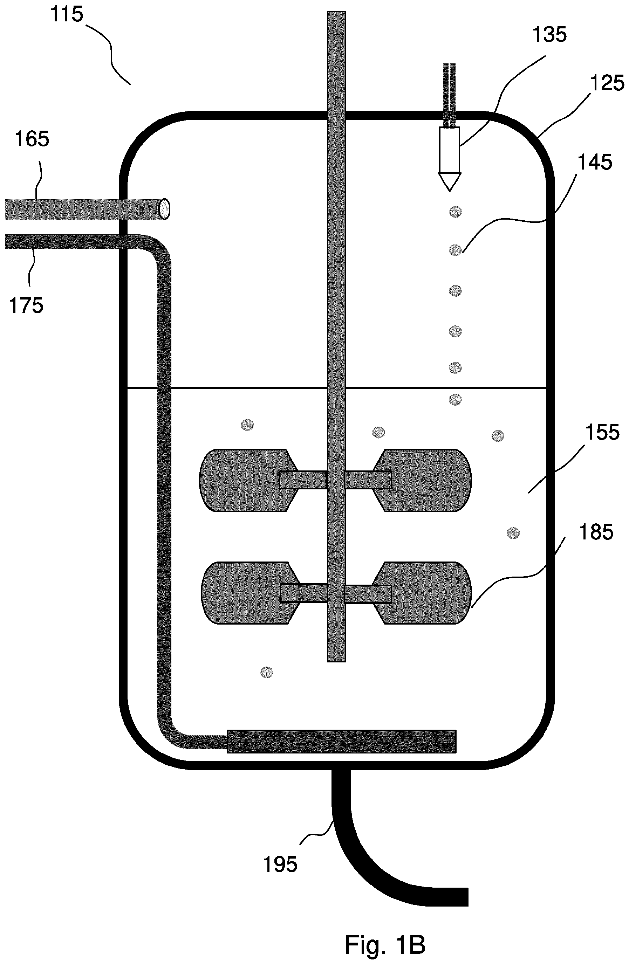

Figure 1 shows (A) a simplified process diagram for a generic bioprocess according to embodiments of the present disclosure, and (B) a schematic representation of a system for implementing a generic bioprocess according to embodiments of the present disclosure;

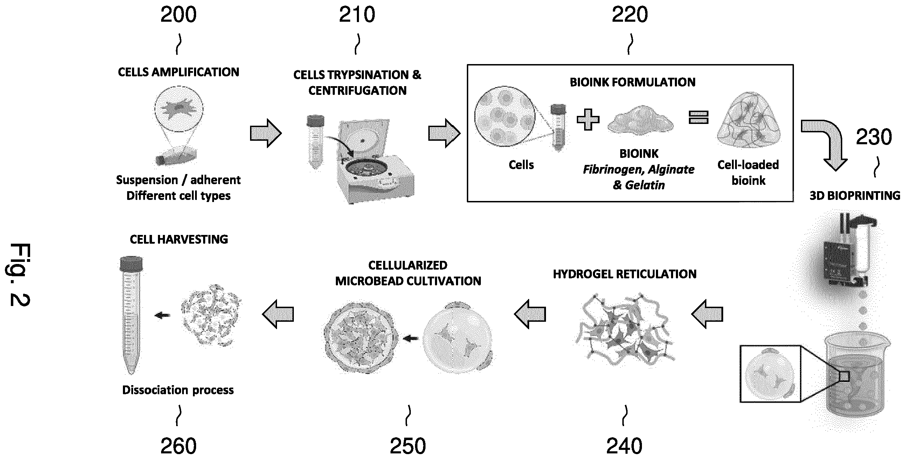

Figure 2 illustrates schematically a process for production of cellular products according to embodiments of the present disclosure;

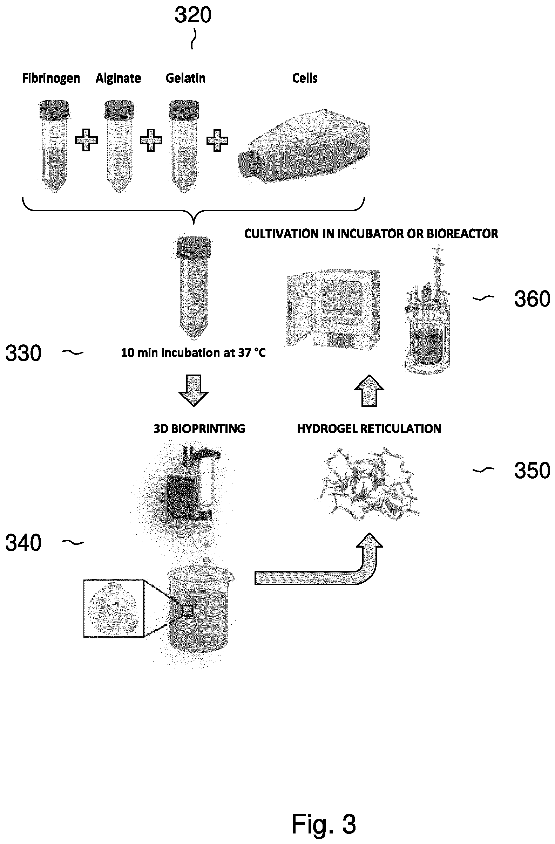

Figure 3 illustrates schematically a process for producing cellularised microcarriers by forming droplets of a bioink, and consolidation of the droplets according to embodiments of the disclosure;

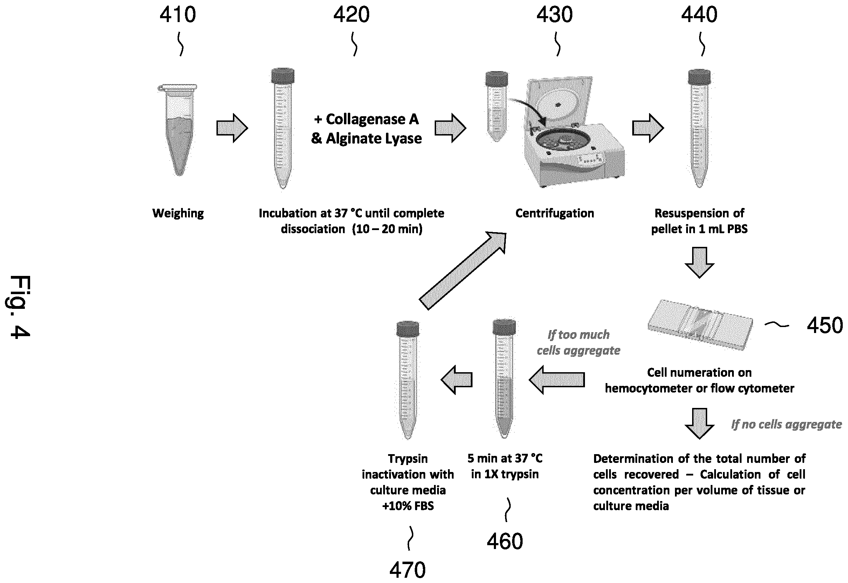

Figure 4 illustrates schematically a process for harvesting of cells from cellularised microcarriers by dissociation, according to embodiments of the disclosure;

Figure 5 shows a graphical representation of cell culture using microcarriers of the prior art (top) or microbeads according to the present disclosure. Using prior art microcarriers, cells are seeded at low numbers and adhere to the surface of the structure, proliferating to cover the surface of the structure. Using microcarriers of the present disclosure (also referred to as "microbeads"), cells are cultured both on the surface of the structure and embedded within it, enabling a microbead to support the growth of a higher number of cells compared to a microcarrier of equivalent size;

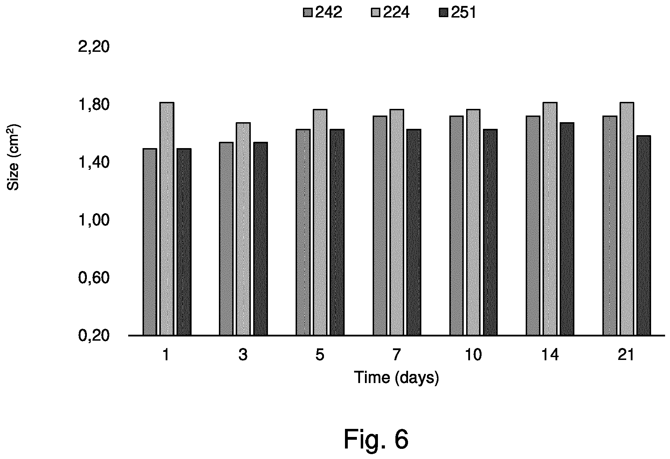

Figure 6 shows the results of an experiment in which the size of manually molded hydrogel balls was monitored, to investigate whether they undergo degradation. The barplot shows the surface area of hydrogel cylinders made of FAG 242, 224, and 251 (left, middle and right bar of each bar group; see Examples section for composition details) over time;

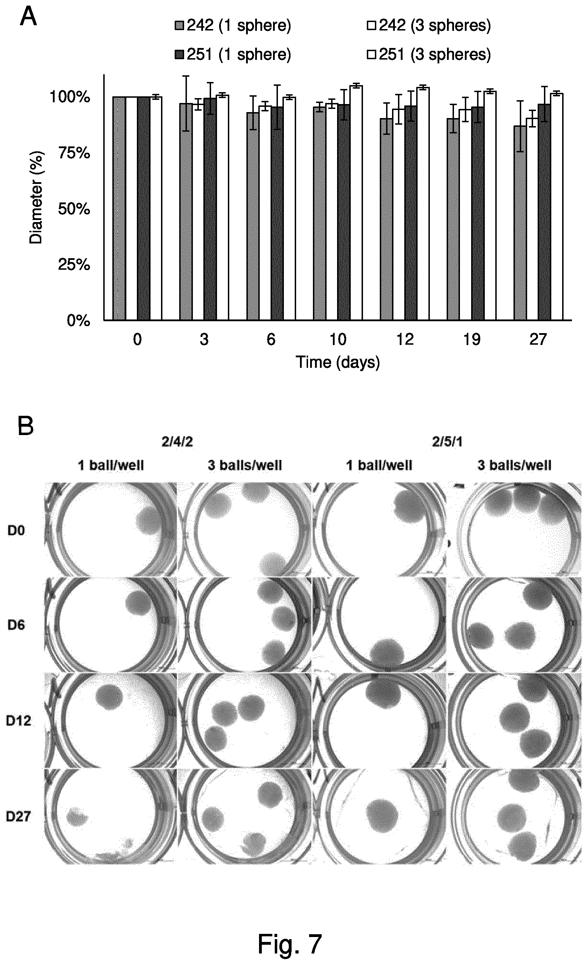

**Figure 7** shows the results of an experiment in which the size of manually cast hydrogel spheres incubated in conditioned culture medium was monitored, to investigate whether they undergo degradation. A. Shows the diameter of hydrogel spheres made of FAG 242 and 251, B. Macroscopic images of the hydrogel spheres used for diameter measurements;

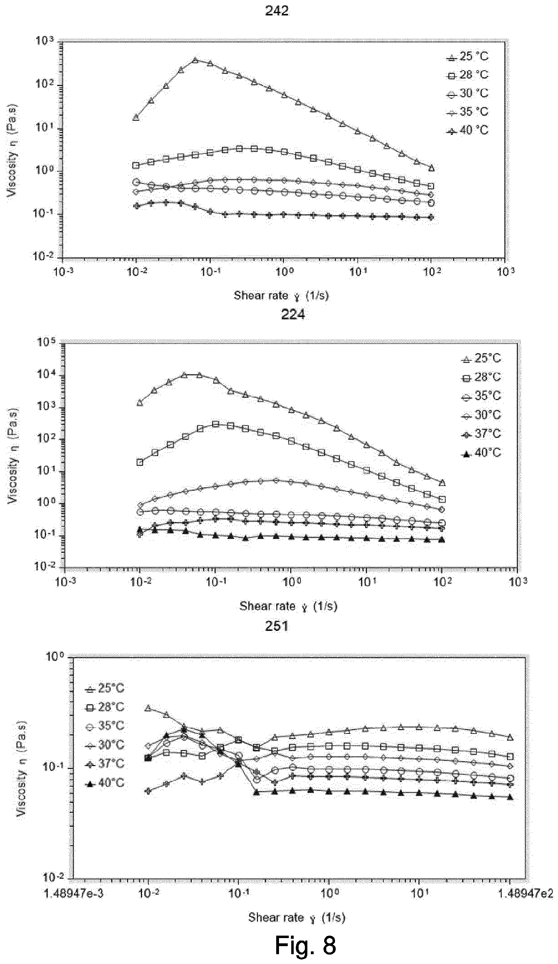

**Figure 8** shows rheograms (viscosity as a function of shear rate) for three bioink compositions (FAG 242 (top), FAG 224 (middle), and FAG 251 (bottom)), measured at different temperatures;

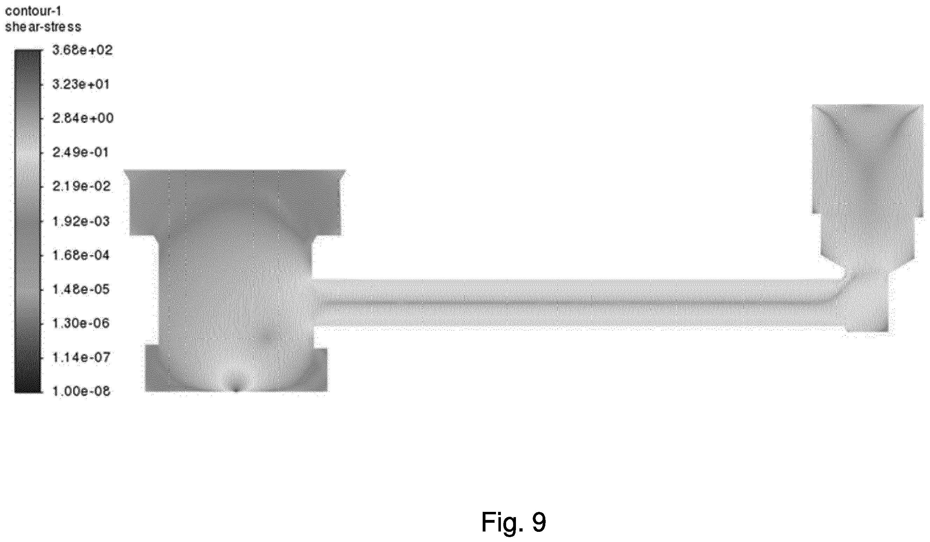

**Figure 9** shows the results of a simulation modelling the shear stress experienced by the bioink inside the inkjet device. The plot shows the intensity of shear stress (Pa) contour for jetting at a pressure of 1 bar. The simulation assumed a viscosity of 0.081 Pa.s, a fluid density of 1035 kg/m$^3$, and a laminar flow model;

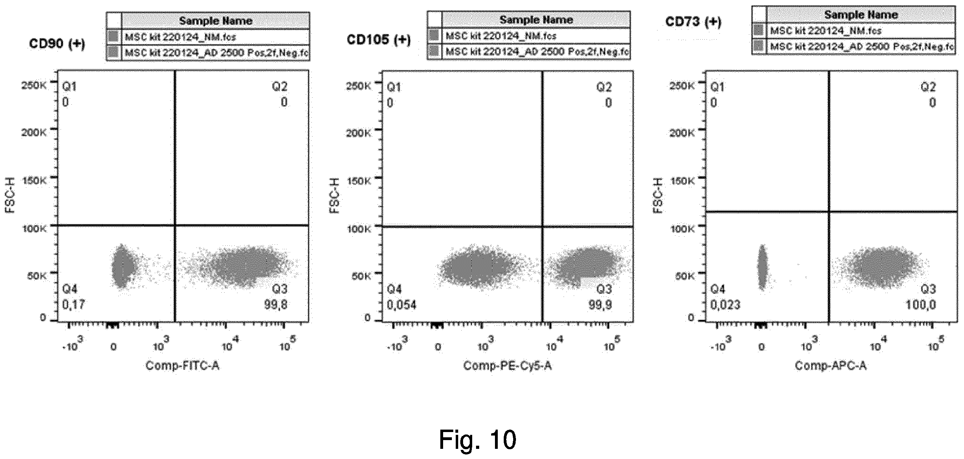

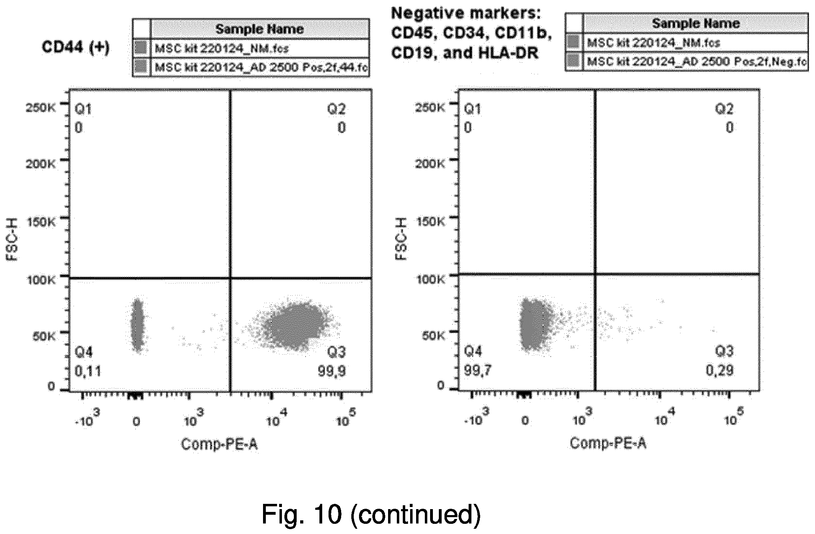

**Figure 10** shows the results of an experiment in which AD-MSCs, cultured in standard conditions, were analysed by flow cytometry to investigate the expression of positive stemness markers: CD90, CD105, CD73, and CD44; and negative markers: CD45, CD34, CD11b, CD19, HLA-DR;

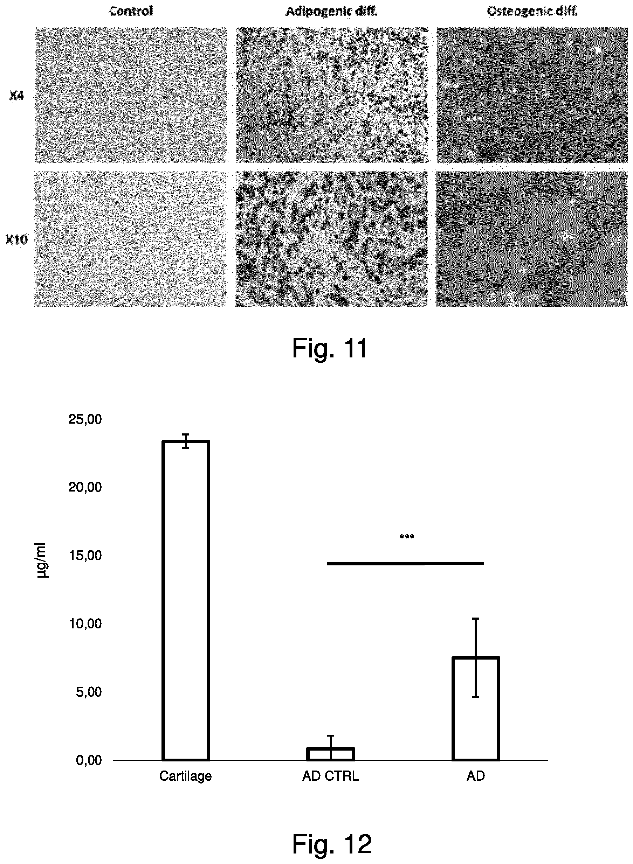

**Figure 11** shows the results of an experiment in which AD-MSCs were cultured in adipogenic or osteogenic differentiation conditions, to investigate their ability to differentiate into these cell types. AD-MSCs cultured in the absence of differentiation conditions were used as a control;

**Figure 12** shows the result of an experiment in which AD-MSCs were cultured in chondrogenic differentiation conditions, to investigate their ability to differentiate into this cell type. The production of glycosaminoglycans (GAGs) was measured as a read out of differentiation. GAG content in AD-MSCs cultured in the absence of differentiation conditions were used as a negative control, and GAG content of native cartilage was used as a positive control;

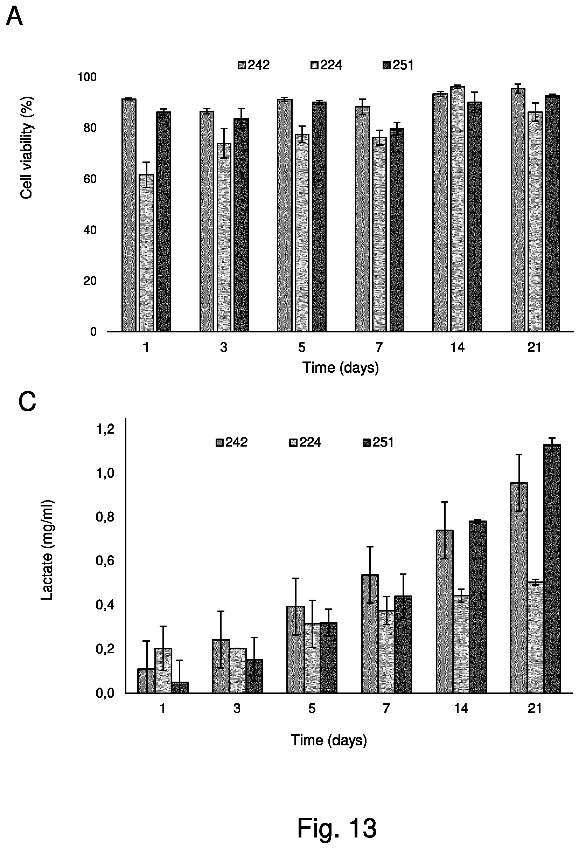

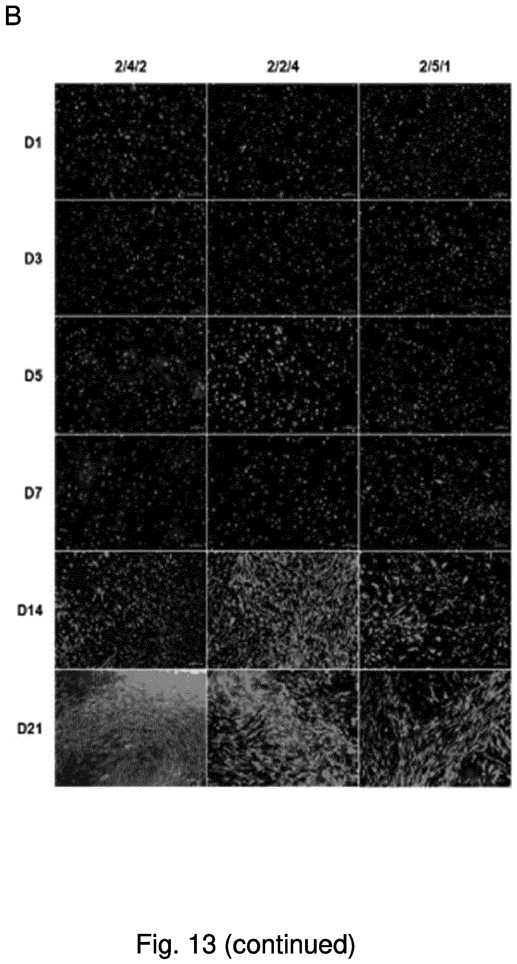

**Figure 13** shows the results of an experiment in which AD-MSCs were cultured in manually casted FAG 242, 224, or 251 hydrogels to investigate cell viability. A. Cell viability rates of AD-MSCs cultured in the different bioink formulations, measured up to day 21 of culture B. Images of AD-MSCs cultured in hydrogels, and stained with a Live/Dead cell viability kit. Live cells are labelled in green, and dead cells in red. C. Cumulative lactate concentration measured for AD-MSCs cultured in each of the bioink formulations, measured up to day 21 of culture;

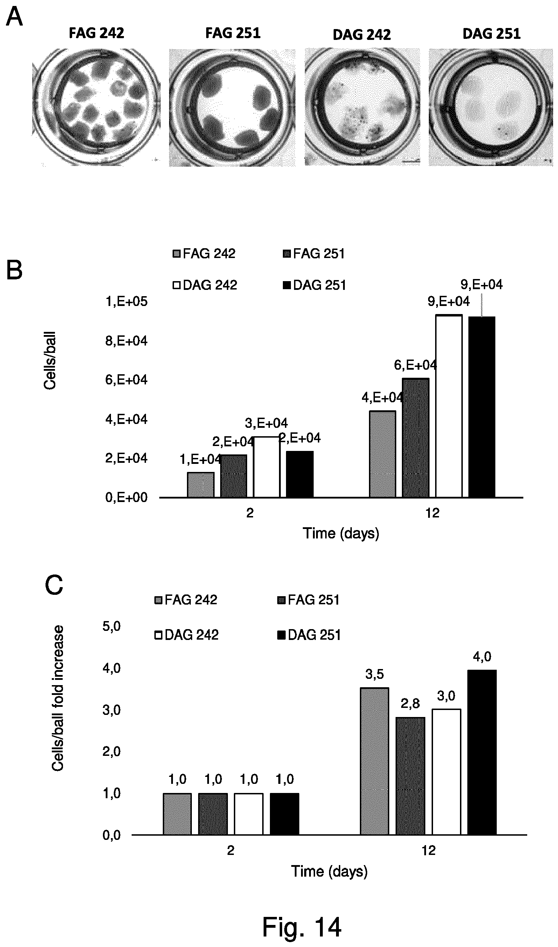

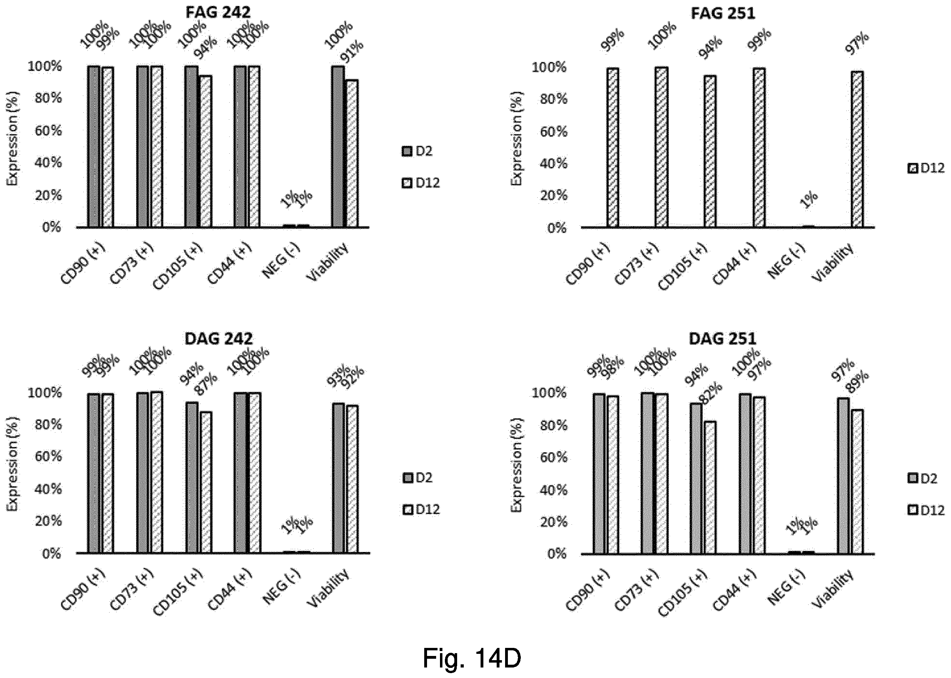

**Figure 14** shows the results of an experiment in which AD-MSCs were cultured in manually cast FAG 242, 224, or 251 hydrogels to investigate cell proliferation and differentiation. A. Macroscopic images of hydrogels made from each of the different bioink formulations, B. quantification of the number of AD-MSCs in the hydrogel samples at 2 and 12 days of culture, C. fold increase of AD-MSCs in the hydrogels at 2 and 12 days, D. Expression of cell surface markers of stemness in AD-MSCs cultured in hydrogels at 2 and 12 days, measured in AD-MSCs isolated from the hydrogels by dissolution;

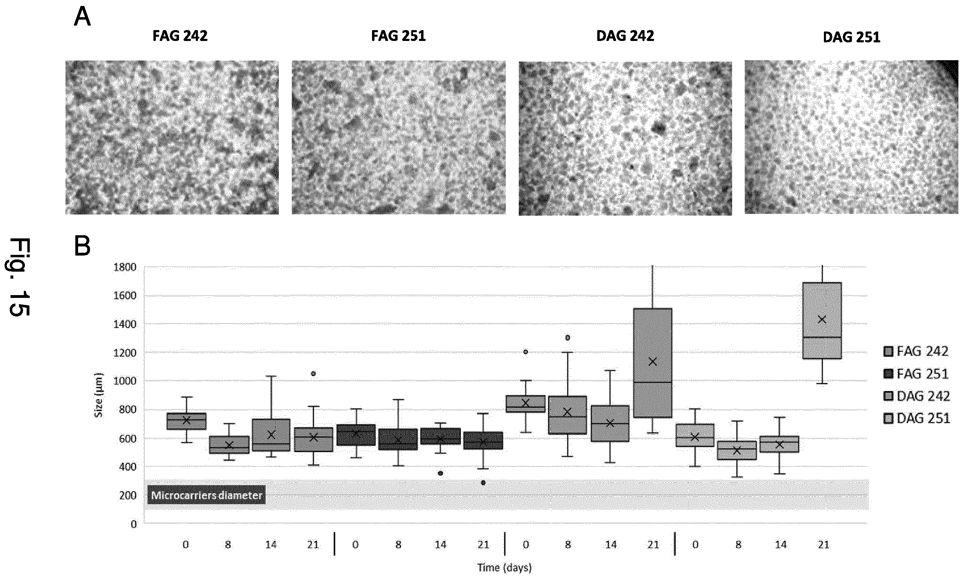

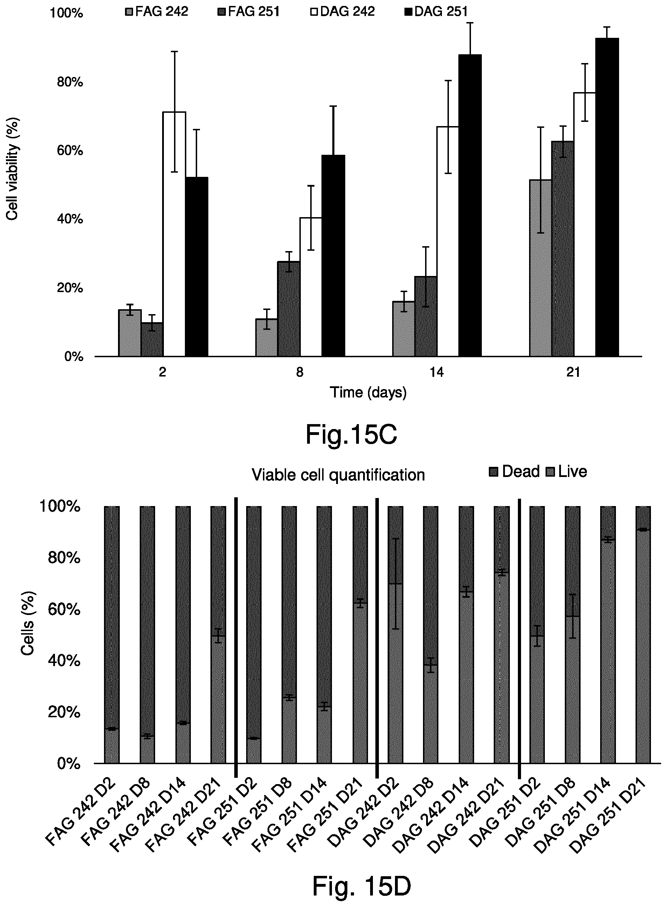

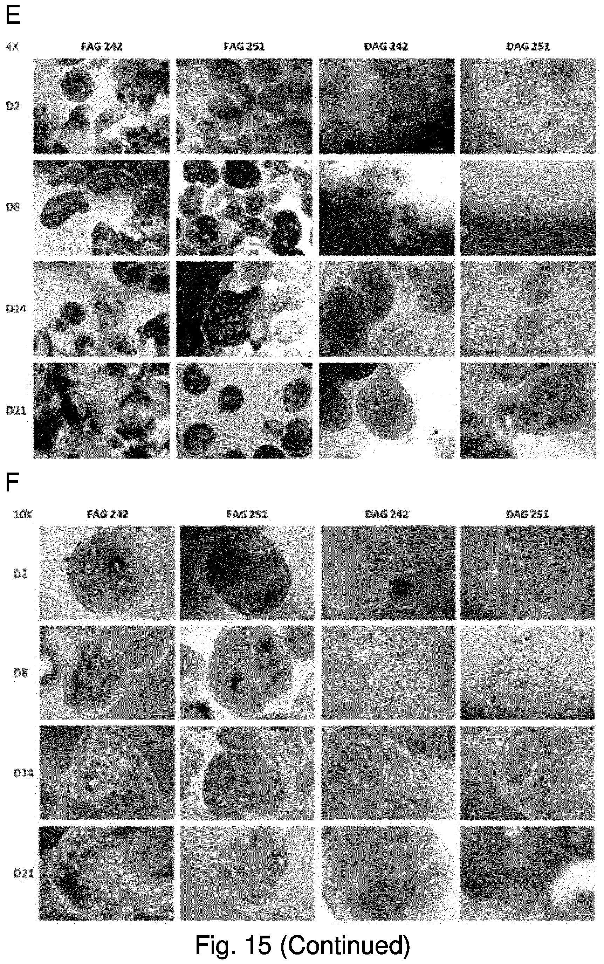

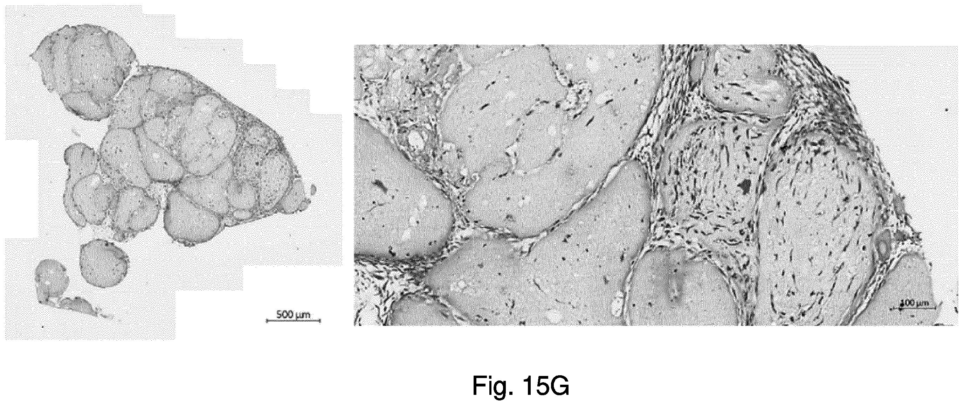

**Figure 15** shows the result of an experiment in which hydrogel microbeads loaded with AD-MSCs were produced by ink-jetting four different bioink formulations through a 100 $\mu$m nozzle. A. Macroscopic images of microbeads produced using FAG 242 and 251, and DAG 242 and 251 bioink formulations, B. Size ranges of the microbeads measured on days 0, 8, 14 and 21 of culture, C. Percentage of viable cells in microbeads of the different formulations, measured on days 2, 8, 14 and 21 of culture, D. cell viability plotted as a live/dead proportion for each of the different formulations, E. Images of the microbeads with live cells stained with calcein (shown in green) and dead cells stained with ethidium homodimer (shown in red), taken at 4X or F. 10X magnification, G. Hematoxylin and eosin staining of an aggregate of microbeads made from the DAG 242 formulation, after 21 days in culture; and

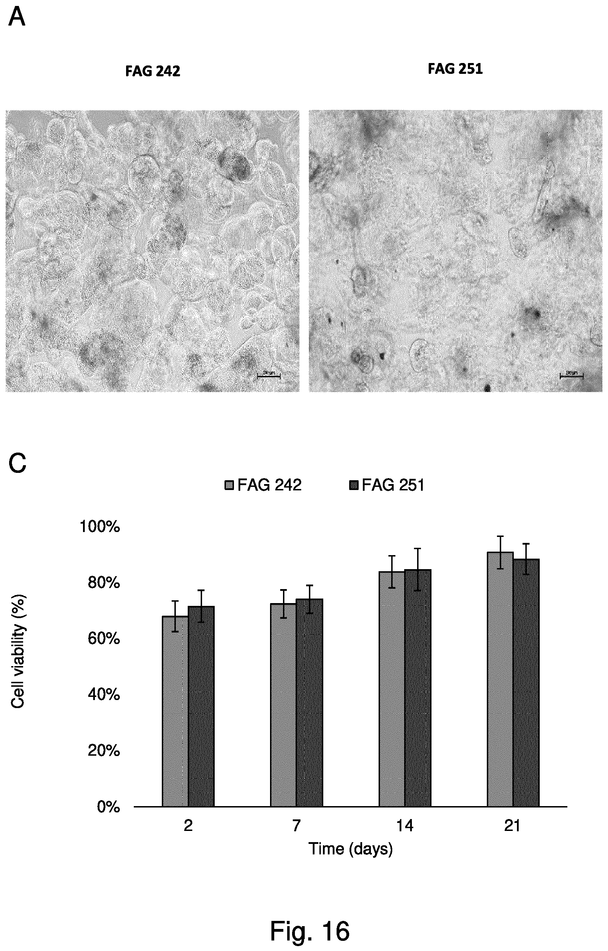

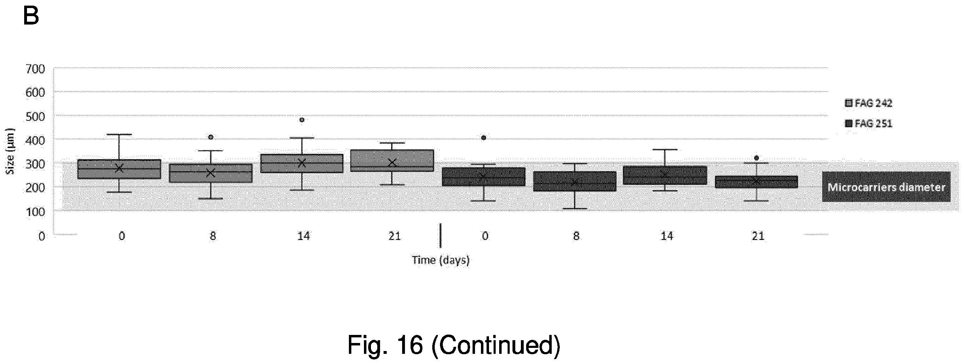

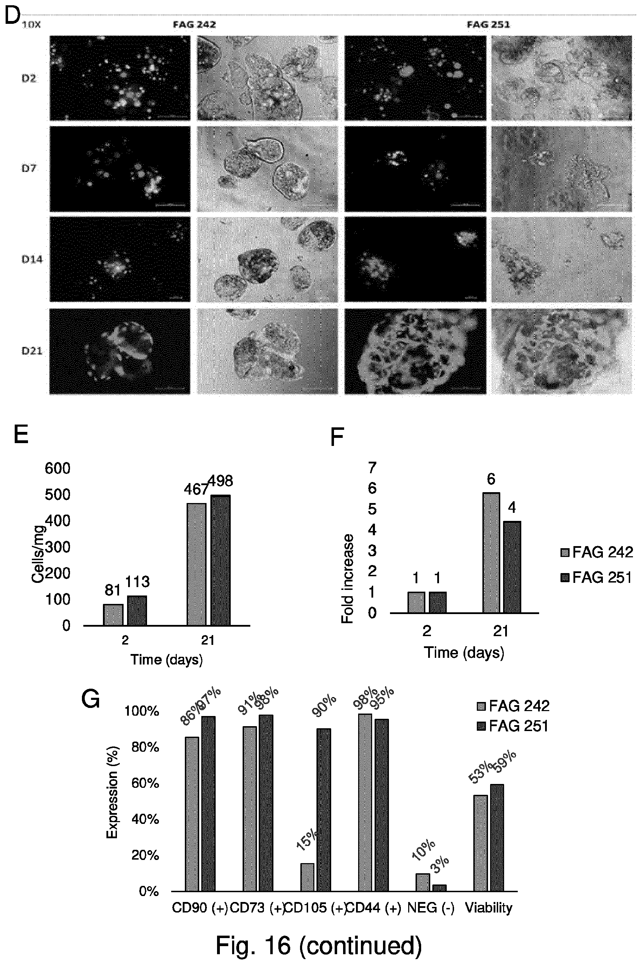

**Figure 16** shows the result of an experiment in which hydrogel microbeads loaded with AD-MSCs were produced by ink-jetting two different bioink formulations through a 50 $\mu$m nozzle. A. Macroscopic images of the microbeads produced using FAG 242 and 251 bioink formulations, B. Size ranges of the microbeads measured on days 2, 7, 14 and 21 of culture, C. Percentage of viable cells in microbeads of the different formulations, measured on days 2, 7, 14 and 21 of culture, D. Images of the microbeads taken at the same timepoints with live cells stained with calcein (shown in green) and dead cells stained with ethidium homodimer (shown in red), E. and F. showing, respectively, quantification of AD-MSC numbers and fold increase of AD-MSCs on days 2 and 21 of culture, G.

Expression of cell surface markers of stemness in AD-MSCs cultured in hydrogels, measured in cells isolated from the hydrogels by dissolution.

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.

The present disclosure relates to microcarriers comprising a particle formed from a continuous hydrogel matrix and one or more cells embedded in the volume of the hydrogel matrix particle, wherein the microcarrier is formed by propelling droplets of bioink by ink-jetting into a liquid medium.

Microspheres formed by dispensing droplets of a composition comprising an extracellular matrix and cells are described in WO2008075206A2, and the related publication Wong et al; Wong, H. L. et al, A 3D collagen microsphere culture system for GDNF-secreting HEK293 cells with enhanced protein productivity. Biomaterials, 28 (35): 5369-5380 (2007). The documents describe microspheres comprising cells and a collagen matrix, which can be used as 3D microcarriers, formed by depositing 2.5 $\mu$l droplets onto a surface, at a temperature of 4 °C. By contrast, droplets according to the present disclosure are dispensed by ink jetting into a liquid consolidation medium. Using the inkjet system enables droplets to be dispensed rapidly, at a high flow rate, which is better suited to large scale cell culture applications. Ink jetting enables the production of substantially smaller microcarriers compared to those described in the prior art, which improves nutrient diffusion into the microcarriers and consequently cell viability. Furthermore, the methods of the present disclosure are performed under conditions optimised for preserving high cell viability (such as e.g. by not requiring the bioink composition to be refrigerated - which increases shear stress during droplet formation), which could not be achieved to the same extent using prior art methods. Using an inkjet system to produce the microcarriers further enables the size of the droplets to be selected for a particular application, for example by changing the nozzle size, and to be better controlled. The inkjet system is also much easier to sterilise compared to e.g. microfluidic systems, due to the modular components that form the flow path. Finally, the inkjet system preserves high viability compared to e.g. microfluidic devices which are known to subject cells to a high amount of shear stress due to laminar flow through narrow channels.

US11511254B2 describes microcarriers comprising a hollow hydrogel shell wherein cells are grown on the internal surface, and methods for their production. The microcarriers are made by concentric extrusion using a co-axial microfluidic device, before consolidation of the outer surface of the shell in an aerosol, and recovery of the microcarriers in a bath. Because the cells only grow on the internal face of the hollow shell, the space which the cells can colonise is limited, and the microcarriers do not utilise the volume of the microcarrier. These microcarriers are produced using a microfluidic apparatus, which is distinct from the ink jetting system of the present disclosure, and associated with a number of drawbacks (as described above).

US2022235320A1 describes dissolvable microcarriers made from polygalacturonic acid cross-linked by a calcium solution. These microcarriers are coated in collagen or Synthemax® to promote cell adhesion, and can be dissolved using a solution of EDTA and pectinase. These microcarriers do not protect cells from shocks caused by bead-to-bead collisions, or shear stress caused by eddy currents in agitated culture systems. Furthermore, they do not maximise the growth capacity of the microcarrier, as cells are only grown on the external surface, rather than throughout the entire volume.

A "microcarrier" is a 3-dimensional structure that supports the growth of adherent cells in bioreactors. The term "microcarrier" is used interchangeably herein with the term "microbead", as the microcarriers of the present disclosure are obtained from liquid droplets and therefore have approximately spherical shapes. Instead of requiring a 2-dimensional surface for cells to grow and adhere to, microcarriers provide a surface that can be maintained in suspension in a liquid medium occupying substantially the entire volume of the bioreactor.

Microcarriers according to the present disclosure comprise a hydrogel matrix. The term "hydrogel" refers to a crosslinked hydrophilic polymer that does not dissolve in water. A hydrogel is a material capable of absorbing a large amount of water. A hydrogel may be a biphasic material, that is, a hydrogel may be a mix of a porous permeable solid and an interstitial fluid. The hydrogel may comprise at least 10% interstitial fluid by weight or volume. The porous permeable solid is insoluble, and may comprise a polymerised molecule. The porous permeable solid may be modified with further molecules to promote cell adhesion. The microcarriers according to the present disclosure are made from a bioink. According to the present disclosure, a bioink is a composition comprising one or more biomaterials that can form a hydrogel, for example after consolidation in appropriate polymerisation condition. The term "bioink" is used interchangeably herein with the terms "bioink solution" and "bioink composition". A biomaterial is a biocompatible material, i.e. a material that is compatible with the maintenance of living cells. In embodiments, the one or more biomaterials that can form a hydrogel comprise alginic acid or a sodium salt thereof also referred to as "sodium alginate" or "alginate" (a naturally occurring polysaccharide which can form a polymer called "alginate" in the presence of e.g. calcium ions, a process also called "gelation"), fibrinogen (a naturally occurring glycoprotein complex which can form a polymer called "fibrin" by enzymatic conversion by thrombin, a process also referred to as "coagulation"), and/or gelatin (a peptide composition that can form a hydrogel when hydrated and maintained at temperatures below gelling point). In embodiments, the one or more biomaterials that can form a hydrogel comprise chitosan. For example, the one or more biomaterials that can form a hydrogel may comprise chitosan, alginate and fibrinogen. As another example, chitosan may be the only biomaterial capable of forming a hydrogel that is used in the bioink. At least one of the biomaterials is such that the microcarriers are stable in the desired cell culture conditions. For example, when the cell culture conditions comprise a temperature T, at least one of the biomaterials is such that it remains polymerised at temperature T. As another example, when the cell culture conditions comprise a concentration of one or more components (such as e.g. ions) within a certain range, at least one of the biomaterials is such that it remains polymerised in media having said composition. As another example, when the cell culture conditions require a pH within a certain range, at least one of the biomaterials is such that it remains polymerised in media having a pH within said range. The bioink may comprise one or more polymerizable biomaterials, wherein one or more of the biomaterials used are such that polymerisation is not reversible. This may increase the stability of the hydrogel in culture conditions. For example, the coagulation of fibrinogen is irreversible, whereas the gelling of alginic acid can be at least partially reversed through removal of calcium ions, and the gel formed by gelatin can be melted by reheating. Other biocompatible materials that can form a hydrogel may be used. The one or more biomaterials may comprise one or more biomaterials that can form a hydrogel, and one or more additional components or additives. The one or more biomaterials capable of forming a hydrogel may comprise or consist of alginate and gelatin. Alginate and gelatin may form the base solution, to which further components may be added. One or more molecules that promote cell adhesion may be added to the base solution (e.g. a solution of alginate and gelatin), such as fibrinogen, Dermial® or hyaluronic acid. Fibrinogen may be consolidated into fibrin through the addition of thrombin. Other components used to promote cell adhesion and which may be added to the base solution include poly-L-lysine and collagen I. Advantageously, in embodiments where the one or more biomaterials capable of forming a hydrogel comprise collagen I, the solution may not comprise a thermal-gelling biomaterial (such as e.g. gelatin). Collagen I may gel at temperatures where agents such as gelatin are soluble, which may make it difficult to control the viscosity of a solution comprising both collagen and gelatin.

In accordance with the present disclosure, a microcarrier is made by propelling a droplet of a bioink solution (also referred to herein as a "bioink composition"). The terms "propelling" in this context may be used interchangeably with "dispensing", "forming" and "ejecting" a droplet. A "droplet" is a volume of liquid formed when force is applied to a liquid, for example, a gravitational force, or an increase in pressure, that causes ejection of the liquid through an aperture. The solution is delivered whilst in liquid from, allowing droplet formation. The process may be referred to herein as "printing" due to the use of an ink jetting device and by analogy to the use of ink jetting devices for 2D or 3D printing (although the droplets are not ejected in a controlled pattern as would be the case when printing). A droplet may be formed by a single ejection event. For example, a droplet may be formed by a single valve opening event, or a single application of a pulse of increased pressure. Thus, the solution may be dispensed directly as individual droplets or may be dispensed as a jet that separates into droplets. For example, liquid jets are known to break up into droplets under certain conditions due to the combination of pressure undulations in the jet and surface tension. Droplets are typically approximately spherical, although departures from perfect spherical shapes are possible and envisaged. An approximately spherical droplet is advantageous to ensure uniform diffusion of nutrients into and out of the droplet. Once a droplet of the bioink solution is formed, it may be consolidated to solidify the 3D structure of the droplet. Consolidation may also be referred to as "cross-linking", "coagulation", "reticulation" or "polymerisation". The bioink solution may be treated with a polymerisation solution (or a plurality of solutions) in order to cause the one or more biomaterials to form a hydrogel. The polymerisation solution(s) may be the liquid medium in which the droplets are collected. The polymerisation solution(s) may be present in a non-static bath in which the droplets are collected. When the bioink comprises alginic acid, the polymerisation solution(s) may comprise calcium ions. When the bioink comprises fibrinogen, the polymerisation solution(s) may comprise thrombin. In embodiments, the polymerisation solution(s) may comprise transglutaminase (TAG). Transglutaminase may be used to cross-link gelatin, resulting in non-reversible consolidation. Gelatin can instead or in addition be consolidated in a reversible manner by changing the temperature of the composition comprising gelatin. When the polymerisation solution(s) are not the same as the growth medium in which the cells are to be cultured, the consolidated droplets (i.e. the cellularised microcarriers) may be washed before being dispensed into the growth medium to be used for growth culture for example inside a bioreactor. The consolidated droplets may be washed in a washing solution such as e.g. PBS (phosphate buffer saline), NaCl 0.9%, or any other buffer compatible with cell viability and integrity of the consolidated droplets. A washing step may be particularly advantageous when the polymerisation solution comprises one or more agents that are advantageously removed prior to cell culture. For example a washing step may be used when the polymerisation solution comprises calcium, thrombin, and/or transglutaminase. In embodiments, a washing step may be used when the polymerisation comprises calcium. A washing step may remove the polymerisation agent(s) (such as e.g. Calcium), thereby reducing the risk of deposit formation after consolidation.

Alternatively, the consolidated droplets may not be washed before being transferred from the polymerisation solution to the growth medium in which the cells are to be cultured. In embodiments, the consolidated droplets are not washed even when the polymerisation solution is different from the growth medium in which the cells are to be cultured. In embodiments, the consolidated droplets may be transferred to the growth medium in which the cells are to be cultured by combining the polymerisation solution containing the consolidated droplets and a growth medium. The terms "microcarrier" or "consolidated droplet" may also be used to refer to a droplet of bioink solution that has been consolidated to form a solid 3D hydrogel structure.

The composition may comprise one or more biomaterial components in such amounts that result in the bioink solution having a low viscosity during jetting, prior to the consolidation of the biomaterials into a hydrogel. A low viscosity may refer to a viscosity below 1 Pa.s, such as e.g. between 0.05 and 1 Pa.s, between 0.05 and 0.9 Pa.s, between 0.05 and 0.8 Pa.s, or between 0.05 and 0.7 Pa.s. The bioink composition may show "shear thinning" behaviour, wherein the viscosity of the solution decreases when a shear force is applied. Alternatively, the solution may show "Newtonian" behaviour, wherein the viscosity remains unchanged when a shear force is applied. Preferably, the composition has a Newtonian behaviour. This ensures that the viscosity of the solution remains constant regardless of the shear stress that is present. The type of fluid and its rheological properties can be assessed using a rheometer, as demonstrated in the examples. The rheological properties of the bioink and the associated conditions for ink jetting that enable the bioink solution to maintain a low viscosity while being compatible with cell viability may be assessed for example as described in the examples. The one or more biomaterials may contribute to the bioink solution having a viscosity sufficient for the composition to be jetted. A bioink with a viscosity sufficient to be jetted may have a viscosity of at least 0.05 Pa.s. For example, the bioink may comprise a gelling agent, such as e.g. gelatin. The amount of a gelling agent such as e.g. gelatin may be used to modulate the viscosity of the bioink solution. The physical conditions during printing may also be modulated to control the viscosity of the bioink solution, keeping it within a desirable range. For example, the bioink composition may be maintained at a temperature between 30C and 40C during printing. Examples of physical conditions that may be modulated during printing, and how to optimise these for making microcarriers by propelling a droplet of a solution, are described in the examples. The rheological properties of the bioink solution may be selected to reduce the shear stress experienced by cells during the printing procedure, or to ensure it remains below a predetermined threshold, for example to maintain cell viability. "Shear stress" is a type of stress that results from the movement of fluid alongside a solid static boundary. The shear stress increases with the flow velocity and the proximity to the solid boundary, such that cells may experience a high amount of shear stress when flowing through the narrow channels of microfluidic devices. The inventors used computational modelling to model the shear stress experienced by cells as they travel through the flow path of an inkjet printer, and have demonstrated that it was possible to produce droplets of bioink using an inkjet printer at low shear stress. For example, the shear stress at any point of the flow path of the bioink during printing may not exceed $5.10^3$ Pa. Suitably, the shear stress in the flow path of the bioink during printing may be between about $1.10^{-1}$ Pa and $5.10^3$ Pa.

The terms "Inkjet printing" and "ink jetting" are used interchangeably herein to refer to the process of propelling droplets of a liquid composition (ink). An "inkjet printer" as used herein refers to a device configured to propel droplets of liquid. Ink jetting may be achieved using a pressure microinjector, such as a syringe, or a pressurised capillary system, or a jetting device comprising a piezoelectric actuation system. For example, an inkjet printer may comprise a liquid tank and a piezoelectric material that changes shape when a voltage is applied, thus enabling a pulse of pressure when a pulse of voltage is applied. The inkjet printer may comprise a jetting valve. The jetting valve may be a piezoelectric jetting valve. A piezoelectric jetting valve may enable jetting with precise timing and volume control. The inkjet device may be a Pico P$\mu$lse™ jetting valve (Nordson), which incorporates a piezoelectric actuation system and provides a modular system where the dispensing nozzle size can be selected between 50$\mu$m and 600$\mu$m diameter, and the dispensing speed can be selected up to e.g. 1000Hz. Droplet deposition is typically controlled electronically, by a computer linked to the inkjet printer. Computerised control may allow the user to adjust the deposition rate, the aperture opening time, and the pressure of the system automatically. In embodiments, an integrated system comprising a bioreactor and an inkjet printer may be controlled centrally by a single computer.

As used herein, the term "bioproduction process" (also referred to herein as "biomanufacturing process" or "bioprocess") refers to a process where biological components such as cells, parts thereof such as organelles or multicellular structures such as organoids or spheroids are maintained in a liquid medium (typically either in suspension or supported on a scaffold) in an artificial environment such as a bioreactor, so that they can perform a function (such as e.g. the production of a biomaterial). The present disclosure relates to bioproduction process that comprise cell culture. A cell culture refers to a bioproduction process whereby live cells are maintained in an artificial environment such as a bioreactor. The methods and systems described herein are applicable to bioprocesses that use any types of cells that can be maintained in culture, whether eukaryotic or prokaryotic. In embodiments, the cells are eukaryotic cells. In advantageous embodiments, the cells are stem cells, and the stem cells themselves are the cell product. A cell line is a population of immortal cells maintained in artificial (culture) conditions. Primary cells are those taken directly from a living tissue (i.e. in vivo) and cultured in vitro. In embodiments, the cells are primary cells. The cells may be animal cells, such as mammalian or insect cells. Preferably, the cells are animal cells that are not insect cells. In embodiments, the cells are mammalian cells. The cells may be embryonic stem cells (ESCs), or adult stem cells (somatic stem cells). The stem cells may be pluripotent or induced pluripotent stem cells (iPSCs). The stem cells may be tissue-specific, such as e.g. epithelial stem cells, skin stem cells, mesenchymal stem cells, or neural stem cells. The cells may be mesenchymal stem cells (MSCs), such as AD-MSCs (adipose-derived mesenchymal stem cells). Stem cells are often susceptible to differentiation during in vitro culture, and it is desirable to suppress this to maintain their pluripotent phenotype. Differentiation refers to the process by which a pluripotent cell acquires characteristic of a particular mature (specialised) cell type. For example, AD-MSCs are able to differentiate into adipocytes, chondrocytes, or osteocytes. A different set of factors including factors selected from transcription factors, chemicals, physico-chemical conditions, and stresses may induce differentiation of different pluripotent stem cells. In embodiments, differentiation may be induced when a cell contacts another cell (known as "contact-induced" differentiation). In embodiments, differentiation may be induced when cells experience shocks and stress during culture (mechanically induced). Therefore, it may be desirable to reduce local cellular density and/or shocks and/or stress during culture of pluripotent cells. The methods of the present disclosure may enable to culture pluripotent cells with reduced local cell density / cell-cell contact compared to 2D adherent cultures or spheroid culture, at higher or similar yields. The methods of the present disclosure may enable to culture pluripotent cells with reduced shocks and stresses compared conventional microcarrier culture systems, at higher or similar yields.

The bioink solution may comprise one or more additives. The one or more additives may be selected based on the cell type that is to be cultured. The additives may be selected to obtain a desired cell phenotype. For example, the additives may be selected to ensure the phenotype of the cell product at the culture endpoint is appropriate for an intended application. In embodiments, additives are included in the bioink solution to prevent or reduce differentiation of the cell population (such as e.g. growth factors such as e.g. FGF2 (also known as FGF-$\beta$), Ginsenoside Rg1, complex mixtures such as serum extracts, or human platelet lysate), in particular when the cells to be cultured are pluripotent cells such as stem cells. The one or more additives may comprise one or more additives that increase cellular proliferation and/or viability (such as e.g. nutrients, vitamins, growth factors, etc.). The one or more additives may comprise one or more additives that alter the rheology of the bioink (e.g. a surfactant), such as e.g. altering its viscosity and/or reducing sedimentation of the bioink droplets in the liquid in which they are cultured and/or consolidated. The one or more additives may comprise one or more additives that increase cellular adhesion in the consolidated droplets (such as e.g. a fibrous biomaterial). The one or more additives may be selected from: Ginsenoside Rg1, L-proline, a surfactant (for example pluronic), one or more growth factors, vitamins (for example ascorbic acid), and fibrous biomaterials (for example, nanocellulose). Ginsenoside Rg1 is a natural molecule found exclusively in the plant genus Panax (ginseng), that regulates various pathways in mesenchymal stem cells and other cells including differentiation, proliferation, apoptosis and senescence. L-Proline is an amino acid that has been shown to improve cell viability and stemness of canine AD-MSCs. Ascorbic acid (vitamin C) is an antioxidant that promotes MSC proliferation while preserving their differentiation capacity. Nanocellulose is a fibrous biomaterial that may be added to the bioink solution to promote cell adhesion. In embodiments, additives may instead or in addition be added to the growth medium in which the microcarriers are suspended. The growth medium in which the microcarriers are suspended may comprise the same one or more additives used in the bioink solution. The growth medium in which the microcarriers are suspended may comprise calcium chloride. Calcium may be included in the culture medium in which the cells are cultured to prevent differentiation of pluripotent stem cells and/or promote proliferation of pluripotent cells (such as e.g. mesenchymal stem cells). In embodiments, the growth medium in which the microcarriers are suspended does not comprise a surfactant, such as e.g. pluronic. Pluronic (also known as poloxamer) is a polymer that behaves as a polymer surfactant due to its hydrophobicity profile. Pluronic is used to reduce shear stress in agitated bioreactor cultures. In the methods of the present disclosure, the cells are embedded within the hydrogel core of the microcarrier, such that they are protected from shear stress within the bioreactor, and advantageously surfactants such as pluronic may not be required.

The bioink solution may comprise one or biomaterials capable of forming a hydrogel, and a buffer or a cell culture medium. A culture medium (also referred to as "growth medium") is a solution designed to support the growth of a population of cells. Growth medium is typically sterile, and comprises substances required for the growth of cells, such as growth factors, and buffering agents. The culture medium used to prepare the bioink solution may be a calcium free growth medium, or a growth medium with low calcium concentration (such as e.g. below 1 mM). For example it may be calcium free DMEM, herein referred to as DMEM(-). A buffer is a solution comprising one or more salts. The use of buffers or culture media may advantageously provide the bioink solution with an osmotic pressure compatible with cell viability. Thus, the bioink solution may comprise a solution that preserves the osmotic pressure of the cells in the bioink solution. For example, the bioink solution may have a NaCl content between 0.2% and 5% w/v. Preferably, the solution that preserves the osmotic pressure of the cells in the bioink solution is a nutritive solution (i.e. a cell culture medium). Examples of culture media are known in the art and include, DMEM, RPMI 1640, MEM, F-12 K, etc. The growth medium may be a growth medium formulated specifically for stem cells, such as e.g. DMEM, MSC GROWTH 2, or MSC NutriStem™ XF. In embodiments, the culture medium does not comprise additives used to protect the cells from hydrodynamic damage, such as detergents. The culture medium may be the same culture medium used to prepare the bioink. In embodiments, the bioink solution comprises between 1 to 2.5% alginate in growth medium. In embodiments, the bioink solution comprises 1 to 10% gelatin in growth medium. In embodiments, the bioink solution comprises 0.5 to 2% fibrinogen, and/or 0.5 to 2% Dermial, and/or 0.5 to 2% hyaluronic acid in growth medium. The precise culture medium used may depend on the cells to be cultured.

The bioink may be consolidated using a polymerisation solution. A polymerisation solution is a solution comprising one or more polymerisation agents, such as e.g. salts (e.g. Calcium salt), or enzymes (e.g. thrombine, transglutaminase (TAG)). Polymerisation agents are agents that cause the polymerisation (also referred to herein as "consolidation", "reticulation", "cross-linking" or "coagulation") of the one or more biomaterials capable of forming a hydrogel. When the bioink comprises alginate and fibrinogen, the polymerisation agents may comprise calcium ions and thrombin. When the bioink comprises gelatin, the polymerisation agents may comprise transglutaminase. The polymerisation solution may comprise at most 5% w/v of a calcium salt, such as between 1% and 5% w/v of a calcium salt (such as e.g. 3% w/v CaCl2). The polymerisation solution may comprise at most 40 U/mL of thrombin, such as between 2 and 40 U/mL (such as e.g. approximately 10 U/mL) of thrombin. The polymerisation solution may comprise at most 4% w/v transglutaminase, such as between 0.5% and 4% w/v transglutaminase. For consolidation, the bioink droplets may be incubated in the polymerisation solution for a predetermined period of time (such as e.g. 10 minutes), for example by immersion of the microcarriers in the polymerisation solution. As described further herein, in embodiments where the droplets are not separated from the polymerisation solution (e.g. no washing step), the droplets may effectively be incubated in the polymerisation solution for as long as the droplets are maintained in culture (or until the cell culture medium has been completely replaced). Thus, the droplets may be incubated in the polymerisation solution for at least a predetermined period of time (such as e.g. at least 10 minutes). Immersion of the microcarriers in the polymerisation solution may be achieved by jetting the droplets directly into the polymerisation solution. Thus, the step of jetting the droplets into a liquid medium may comprise jetting the droplets into a solution comprising one or more polymerisation agents. The polymerisation agents may be selected depending on the biomaterials in the bioink solution. Immersion of the microcarriers in the polymerisation solution may advantageously result in homogeneous polymerisation of the biomaterials. The polymerisation solution may comprise a growth medium. In other words, the growth medium may comprise one or more polymerisation agents. Thus, the polymerisation of the droplets and culture of the cellularised microcarriers may be performed in the same solution. A polymerisation solution may comprise a cell culture medium and one or more polymerisation agents. The cell culture medium may be the same or different from the cell culture medium used to prepare the bioink solution and/or to culture the cells.

The bioink composition may be prepared for use from one or more stable aqueous solutions, also referred to as "stock solutions". For example, the bioink composition may be prepared using one or more of: a first solution comprising between 5% and 40% w/v (weight by volume) of gelatin (such as e.g. approximately 20% w/v of gelatin), a second solution comprising between 1% and 12% w/v of alginate (such as e.g. approximately 4% w/v of alginate), and a third solution comprising between 1% and 15% w/v of fibrinogen (such as e.g. approximately 8% by mass fibrinogen). The solutions may be mixed in predetermined amounts with a suspension or pellets of cells. When a suspension of cells is used, the suspension may be in a culture medium. When alginate is used as one of the biomaterials, the suspension of cells (and every other solution used to prepare the bioink) is preferably calcium free. For example, a calcium free culture medium such as DMEM(-) may be used to suspend the cells prior to mixing with the other components of the bioink..Culturing cells using the microcarriers of the disclosure may comprise formulating the bioink such that the cells are present at a predetermined cellular density (also referred to as "cell seeding density" or simply "density"). The predetermined cellular density per volume of bioink may be between 1.0E+05 cell/ml and 1.0E+07 cell/ml, or between 5.0E+05 to 5.0E+06. The predetermined density may be between around 1.0E+06 cell/ml and around 2.0E+06 cell/ml. After jetting, the number of cells per droplet of bioink solution may depend on the size of the droplet. In embodiments, the droplet comprises between 1 and 50 cells, between 1 and 40 cells, between 1 and 30 cells, between 1 and 20 cells, between 1 and 10 cells, between 2 and 8 cells, between 4 and 6 cells, less than 50 cells, less than 40 cells, less than 30 cells, less than 20 cells or less than 10 cells. When referring to a plurality of droplets (i.e. a population of droplets), these quantities may refer to the average number of cells per droplet, over the population of droplets. Thus, a plurality of droplets may comprise, on average, between 1 and 50 cells per droplet, or e.g. less than 10 cells per droplet (e.g. on average 3, 4, 5, 6, 7, 8, or 9 cells per droplet). The cells may be cultured until the number of cells per microcarrier (where each microcarrier corresponds to a consolidated droplet) increases between 2- and 30-fold as compared to the starting population, between 2- and 60-fold, between 2- and 100-fold, or between 2- and 125-fold as compared to the starting population. For example, the cells may be seeded at 5 cells/microcarrier, and cultured until the cell number increases 15-fold, such that there are 75 cells/microcarrier at the culture endpoint.

A bioreactor is a device in which a biological component (e.g. a cell or part thereof) can be maintained in controlled environmental and operating conditions (e.g. pH, temperature, pressure nutrient supply, and waste removal). A bioreactor typically comprises at least a vessel suitable for holding a liquid culture medium. According to the present disclosure, a bioreactor may be a stirred tank bioreactor, configured with agitation means for culturing cells (or microcarriers comprising or supporting cells) in suspension. The configuration, shape and/or volume of the bioreactor may depend on the bioprocess. The vessel may be a single use vessel, or a reusable vessel. The vessel may be made primarily of stainless steel, plastic or any other material known in the art for use in industrial bioprocesses. The volume of the tank may be determined based on the requirement of the bioprocess, such as e.g. a desired number of cells at the end of the bioprocess and/or a desired cell seeding density (per volume of liquid culture medium), such as e.g. based on an expected growth and desired number of cells at the end of the bioprocess, and/or a desired number of microcarriers per ml of culture medium in the vessel. For example, it may be advantageous for the number of cells per ml of culture medium in the vessel at the start of the culture to be at least $10^4$ cell/ml (such as e.g. between 1.0E+04 cell/ml and 1.0E+08 cell/ml, preferably at least 1.0E+05 cell/ml) at the start of the culture (i.e. seeding density). As another example, a bioprocess may use at least 10000 microcarrier per ml of cell culture medium, such as e.g. between 10000 and 150000 microcarriers per ml of cell culture medium. The cells in culture may grow to reach cell densities of at least 20 cells / microcarrier, at least 50 cells/microcarrier, at least 70 cells per microcarrier, at most 100 cells per microcarrier, between 20 and 100 cells per microcarrier, between 50 and 100 cells per microcarrier, or about 80 cells per microcarrier. The amplification fold during cell culture using the microcarriers described herein may be at least 10, 20 or 30 fold. For example, cells may be seeded at approximately 3 to 6 cells per microcarrier and may grow to approximately 30 to 120 cells per microcarrier. The amplification fold may dependent on at least the initial cell seeding density per microcarrier and the size of the microcarriers (e.g. volume of the droplet). For example, larger droplets with lower cell seeding densities per droplet may grow to higher amplification folds than smaller droplets with lower cell seeding densities per droplet. The bioreactor may be equipped with one or more fluid inlet(s) and one or more fluid outlet(s). The fluid inlet(s) and outlet(s) may enable the provision of nutrients, gasses etc. to the cells in the microcarriers, by facilitating the exchange of nutrients into and out of the hydrogel core of the microcarrier. The fluid inlet(s) and/or outlet(s) may comprise a filter to prevent the passage of microcarriers. The fluid inlet(s) and outlet(s) may respectively be connected to a supply tank and collection system. The flow at the inlet(s) and/or outlet(s) may be constant or variable during the bioprocess, whether in composition or in volumetric flow. For example, the flow of liquid through the bioreactor (assuming that the volume of liquid flowing into the bioreactor is equal to the flow volume of liquid flowing out of the bioreactor) may be increased one or more times during the bioprocess, to support the growing number of cells.

Stirred tank bioreactors are agitated, or "stirred", to keep the cells and/or microcarriers in suspension, and to prevent their sedimentation. The bioreactor may be agitated by the flow of gas through the growth medium within the bioreactor (air-lift agitation). The bioreactor may be agitated by an impeller. The impeller may be rotary, and may comprise one or more fins. The impeller may be a flat blade turbine or a propeller. The impeller may be configured and/or controlled to ensure homogenous distribution of the microcarriers throughout the growth medium. The impeller may be configured to produce an axial or radial flow of growth medium within the bioreactor. Impellers for use in stirred tank bioreactors are known in the art, and the high shear forces they cause within the growth medium itself are a known problem for the culture of shear-sensitive cells. Impellers and other agitation means cause eddy currents (circular currents flowing in an opposite direction to the main body of fluid) in the growth medium in which the cells and/or microcarriers are suspended. Eddy currents in particular can cause shear stress as they flow past cells and/or microcarriers. The impeller configuration and/or the agitation rate may be configured to reduce the formation of eddy currents, and to reduce the shear stress. The bioink droplets may be jetted directly into a liquid medium (also referred to as a "recovery bath" or "collection bath"). As explained above, the recovery bath may comprise a polymerisation solution. The recovery bath may be agitated using any agitation means known. For example, the recovery bath may be agitated using air-lift, or using mechanical means such as an impeller or a magnetic stirrer. When the agitation means are rotary agitation means (such as e.g. an impeller), they may controlled to ensure that the jetted droplets flow away from the jetting location (e.g. to avoid aggregation of the droplets), while not resulting in accumulation of the droplets on the sides of the vessel through centrifugal force. For example, the speed of the rotary agitation means may be adjusted to ensure that this is achieved. The exact speed that achieves this may depend on the configuration of the vessel and impeller, the composition of the liquid medium, the composition and size of the droplets, and the jetting parameters. The microcarriers of the disclosure may also be used to culture cells by alternative methods. The microcarriers may be used to culture cells on a smaller scale than in a stirred tank bioreactor. The microcarriers may be used to culture cells in spinner flasks, or in flasks which do not comprise an integrated agitation means and are instead placed in a shaker incubator. The parameters (e.g. intensity) of the agitation may be set such that it is high enough to keep the microcarriers in suspension. The intensity / power input of the agitation may be set at or near (e.g. within 10%) of the minimum intensity at which the microcarriers are maintained in suspension. In embodiments, agitation is provided using an impeller rotating at a speed of between 20 rpm to 500 rpm, or a speed of less than 100 rpm. In embodiments, the cell culture is performed in a stirred tank (e.g. a bioreactor such as e.g. Ambr250 by Sartorius) comprising an impeller rotating at a speed of between about 35 rpm and about 60 rpm. The minimum level for the intensity of agitation that keeps the microcarriers in suspension may depend on the size and/or shape of the vessel, and/or the size and/or density of the microcarriers. In embodiments, the intensity of agitation increases over time, e.g. the impeller may reach a maximum speed of rotation of around 60 rpm.

**Figure 1A** shows a simplified process diagram for a generic bioprocess according to the disclosure. The illustrated process comprises optional step 120 of providing a solution (also referred to herein as "bioink composition" or "bioink solution") comprising a cell population and one or more biomaterials capable of forming a hydrogel. The bioink composition is formed into droplets by ink-jetting at step 130. The bioink droplets may then be consolidated at step 140, thereby forming microbeads, also referred to herein as "microcarriers" or "microcarrier particles". At step 150, the microbeads may be cultured, resulting in the growth of the cell population. This may include the step of replacing or supplementing some or all of the culture medium. At step 160, one or more cellular products are harvested. This may comprise separating the cells from the hydrogel matrix, at the end of the culture. Embodiments of these generic steps are described in more detail by reference to Figures 2-4.

**Figure 1B** illustrates schematically a system for implementing a generic bioprocess according to embodiments of the disclosure. The illustrated system 115 comprises a stirred tank bioreactor 125 with an integrated inkjet printer 135, for jetting hydrogel droplets 145 directly into the liquid medium 155 in the bioreactor 125. The inkjet printer 135 as illustrated is located at least partially within the bioreactor (i.e. at least the nozzle of the jetting device is located within the bioreactor to enable jetting directly into the liquid medium within the bioreactor). Alternatively, the inkjet printer may be connected with a recovery tank comprising a liquid medium in which the droplets are jetted. The recovery tank (not shown) may be fluidically connected with the bioreactor 125. The recovery tank may also comprise an agitation means. The inkjet printer is fluidically connected with feed lines 145 for providing the solution of bioink to the printer. Consolidation of the droplets may be performed in the bioreactor (when the droplets are jetted directly in the bioreactor), or in the recovery tank (when the droplets are jetted in liquid medium in a recovery tank). The consolidated hydrogel droplets may undergo a wash step before they are suspended in culture medium for cell culture. The washing step may be performed in the bioreactor, in the recovery tank, or in a device connected to the recovery tank and/or the bioreactor (not shown). Alternatively, the hydrogel droplets may be printed directly into the bioreactor culture medium. The bioreactor shown in the embodiment in Figure 1B may be a standard commercially available bioreactor, comprising an inlet for culture medium 165, an air supply 175, an agitation means 185, and an effluent 195. The system may also comprise further equipment commonly used in stirred tank bioreactors, such as monitoring devices.

**Figure 2** illustrates schematically a process for production of cellular products according to embodiments of the present disclosure. At step 200, a population of cells (typically stem cells) is amplified using conventional cell culture protocols such as e.g. using a suspension culture or adherent culture. The choice of a suitable amplification protocol may depend on the cell type at hand. At step 210, the amplified cell population is harvested, for example by centrifugation and/or trypsinisation (depending on the cell culture protocol used at step 200). At step 220 a bioink is formulated by mixing the harvested cells with a solution comprising biomaterials capable of forming a hydrogel (in particular, gelatin, alginate and fibrinogen, in the illustrated embodiment). At step 230, the bioink composition is 3D printed, by ink jetting in the illustrated embodiment. At step 240, the bioink composition is consolidated. In the illustrated embodiment, the microbead structure is maintained by the gelatin during printing. The gelatin may at least partially dissolve (depending e.g. on temperature in the bioreactor and/or the use of a gelatin cross-linker such as transglutaminase), and a more permanent structure is formed by cross linking the alginate and fibrinogen, respectively. The partial dissolution of the gelatin may create microporosities that advantageously enable further cell growth. At step 250, the cellularised microbeads are cultured in a culture medium, during which the cell population grows to colonise the microbeads. At step 260, the cells are harvested by dissociating the hydrogel matrix.

**Figure 3** illustrates schematically a process for providing cellularised microbeads according to embodiments of the disclosure. At step 320, a solution of cells (such as e.g. cells suspended in culture medium or buffer, preferably low calcium or calcium free culture medium such as DMEM(-) or an MSC culture medium such as MSC GROWTH 2 available from Promocell) is mixed with stock solutions each comprising one or more biomaterials, for example three solutions respectively comprising 10 % w/v gelatin, 4 % w/v alginate and 8 % w/v fibrinogen. Preferably, each of these stock solutions are prepared in culture medium. The solutions may be mixed in predetermined amounts to obtain desired amounts of the biomaterials. For example, the solutions may be mixed to result in a composition comprising 5% w/v gelatin, 2% w/v alginate and 2% w/v fibrinogen (a composition referred to in the examples below as "FAG242"). Note that alternative concentrations of each of these components and compositions comprising two or all three of these biomaterials may be used instead. This solution may be referred to as a bioink. At step 330, the bioink is incubated at 37°C to allow the solution to homogenise and the cells to recuperate, for example for 10 minutes. This may be performed in the same syringe that will be used for printing. Note that the precise times and temperatures provided are indicative only, and alternative times / temperatures may be used depending on the cell population, the composition of the bioink (ingredients and concentrations) and the desired printing viscosity (which can depend on the ink-jetting parameters). The solution may be maintained at a temperature between 30°C and 40°C between steps 320 and step 340, and optionally throughout the printing process (such as e.g. by heating the printer reservoir and optionally the nozzle and/or any other part of the fluid path within the printer). This may ensure that the viscosity of the bioink remains constant and does not increase so as to result in increasing shear stress for the cells during printing. At step 340, the bioink is ejected through the aperture of a nozzle of an inkjet printer to form droplets. The size of the nozzle may be selected depending on the desired size of the droplets. Suitably, the nozzle may have a diameter between 50 $\mu$m and 300 $\mu$m. Smaller nozzles lead to smaller droplets, and ultimately smaller microcarriers. Smaller microcarriers have better diffusion of nutrients and gasses during cell culture, but jetting through smaller nozzles is associated with higher shear stress. The jetting may be performed at a pressure between 0.25 and 5 bars, between 1 and 5 bars, or between 2 and 5 bars. Lower pressures within these ranges may be sufficient for compositions that have low viscosities (e.g. compositions comprising alginate and between 0 and 5% w/v gelatin), whereas high pressures within these ranges may be beneficial for compositions that have higher viscosities (e.g. compositions comprising alginate and between 5 and 10% w/v gelatin). Lower pressures may not result in jetting of the bioink, whereas higher pressures may excessively stress the cells. The pressure that is suitable to obtain a reliable jetting effect may depend on the size of the nozzle. For example, with a nozzle diameter of 50 $\mu$m a pressure of 4 bar may be suitable, whereas with a nozzle diameter of 100 $\mu$m a pressure of 3 bar may be sufficient. At step 350, the droplets are consolidated by incubation with a polymerisation solution, resulting in microbeads or microcarriers suitable for cell culture. The timing of consolidation may depend on the concentration of the polymerisation solution and the size of the microbeads. The temperature of consolidation may depend on the cells and on the composition of the bioink (for example, the temperature may be chosen to be sufficient to at least partially re-melt the gelatin without damaging the cells). Advantageously, the temperature may be maintained between room temperature and 40C, such as e.g. between 20°C and 40°C, to improve cell viability. At step 360, the consolidated cellularised structure is cultured in a bioreactor or other culture vessel.

**Figure 4** illustrates schematically a process for harvesting of cells from cellularised microcarriers by dissociation, according to embodiments of the disclosure. At step 410, the celllularised microbeads are weighed or counted to determine the parameters of the dissolution protocol. At step 420, the cellularised microbeads are incubated in a dissolving solution such as in collagenase and/or dispase and/or papain (advantageously a solution comprising a concentration of collagenase below 3 % w/v , such as e.g. 0.5-5 % w/v, or approx. 4.5 U/ml in order to avoid causing damage to the cells) and alginate lyase in a PBS buffer (advantageously a solution at a concentration of around 0.5 mg/ml, such as e.g. a concentration between 0.1 mg/ml and 2 mg/ml, or approx.. 5U/ml), in the illustrated embodiment. In embodiments, other enzymes may be used instead or in addition to collagenase, such as e.g. dispase and papain. For example, a composition comprising 0.1 U/ml collagenase A and 0.8 U/ml dispase may be used. This may be performed at 37C or any other temperature that is compatible with cell viability and action of the enzyme used for dissolution. The timing of incubation may depend on the size and amount of microbeads, and incubation may be maintained until complete dissociation of the microcarriers. This may take about 2 to 15 minutes. The dissociation solution may then be inactivated as the enzymes may cause damage to the cells. This may involve the addition of one or more enzyme inhibitors, such as foetal bovine serum or a highly concentrated protein solution (alone or as part of e.g. a cell culture medium). These may be added to the dissolving solution or may be used to replace at least part of the dissolving solution. At step 430 the solution is centrifuged to separate the cells from the supernatant comprising the dissolved matrix. At step 440 the cell pellet is resuspended, for example in PBS or culture medium. The volume of solution used may depend on the amount of cells and the subsequent steps to be performed. At step 450, the cells are counted using a sample of the solution obtained at step 440. This may be performed using any method known in the art, for example using a cell counting device such as a hemocytometer or a flow cytometer. Any cell counting protocol known in the art may be used. It is then determined whether the cell solution comprises many cell aggregates. If that is not the case then the result of step 450 enables the determination of the final cell concentration as a result of the bioprocess. If too many cell aggregates are present (where the threshold for this may depend on the particular application), the cells may be briefly incubated with trypsin to break up these aggregates at step 460. This may use trypsin 1x at 37°C for 5 minutes or any other trypsinisation protocol known in the art. Note that this may only be performed if individualisation of the cells is necessary or desirable, but is not necessary for the harvesting of the cells per se. The trypsin may then be inactivated at step 470, as trypsin may cause damage to the cells. This may involve the addition of culture media with foetal bovine serum or other inhibitor solutions. The resulting solution may then be centrifuged (step 430), resuspended (step 440) and re-counted (step 450). The cell counting steps 450 to 470 are optional, and the harvested cell solution obtained at step 440 may be used directly without cell counting.

## EXAMPLES

Exemplary methods of producing cells in hydrogel microcarriers, as well as exemplary methods for obtaining such microcarriers by ink jetting a bioink will now be described.

### *Materials and Methods*

*Cell Lines and Culture.* Adipose-derived mesenchymal stem cells (AD-MSCs) were purchased from the "Banque de tissues et cellules Hopitaux Civil de Lyon" and cultured at 37C in a 5% CO2 atmosphere using MSC GROWTH media (Promocell). When 80% of confluence was reached, cells were released with trypsin (Sigma) and sub-cultured.

Generally, cells were amplified in conventional adherent cultures in flasks (Fig. 2, step 200). The cells were then trypsinised (Fig. 2, Step 210), and formulated as a bioink formulation (Fig. 2, step 220). The formulation was jetted (Fig. 2 step 230), and the construct was consolidated (Fig. 2, step 240). After culture (Fig. 2, step 250), the cellularised microcarriers were dissociated to harvest the cells (Fig. 2, step 260).

*MSCs characterisation.* Phenotype and differentiation potential of AD-MSCs between passages P4 - P6 were characterized before use in accordance with the ISCT criteria for defining multipotent MSCs (Zweigerdt, R. Large Scale Production of Stem Cells. Advances in Biochemical Engineering/Biotechnology 1-35 (2009) doi:10.1007/10). ISCT criteria includes: (a) plastic-adherent growth when maintained in standard culture conditions; (b) > 95% of cells show expression of at least three positive stemness markers, and < 2% expression of negative markers; and (c) the capacity to differentiate towards the three mesodermal lineages (adipogenesis, osteogenesis, and chondrogenesis). Cell surface markers were analyzed using the Human MSC Analysis Kit (BD) by cell cytometry. For adipogenic, osteogenic, and chondrogenic differentiation, AD-MSCs were cultured for 3 weeks in adipogenic, osteogenic, and chondrogenic media (Silva Couto, P. et al. Expansion of human mesenchymal stem/stromal cells (hMSCs) in bioreactors using microcarriers: lessons learnt and what the future holds. Biotechnology Advances 45, 107636 (2020); Badenes, S. M. et al. Microcarrier Culture Systems for Stem Cell Manufacturing. Stem Cell Manufacturing (Elsevier B.V., 2016). doi:10.1016/B978-0-444-63265-4.00004-2.). Differentiated cell cultures were stained with red oil (Sigma) for adipogenic differentiation, alizarin red (Sigma) for osteogenic differentiation, or safranin (Sigma) for chondrogenic differentiation. Since there were issues with the safranin chondrogenic staining, to ensure the chondrogenic differentiation, glycosaminoglycans (GAGs) were quantified using the kit Glycosaminoglycan Assay Blyscan™ (Biocolor). To do so, AD-MSCs spheroids were produced by centrifuging $0.25 \times 10^6$ cells/Eppendorf tube, cultured inside the tubes for 21 days under differentiation conditions, and finally analyzed by cell cytometry.

*Bioink preparation.* Fibrinogen (8%; F8630 Sigma), alginate (4%; A18565 Alfa Aesar), gelatin (20%; G1890 Sigma), and Dermial® (4%; from Bioiberica; 60-75% hyaluronic acid (HA) + >10% Dermatan sulphate (DS) / Chondroitin sulphate (CS) + >5% Collagen) were prepared using DMEM without calcium (Sigma) or MSC GROWTH media 2 (Promocell), and allowed to fully dissolve overnight at 37C inside an incubator. The media was supplemented with 0.4% Amphotericin B (Gibco) and 0.5% Penicillin-Streptomycin (Gibco). Cells were added to the fibrinogen or Dermial® solutions, then combined with alginate, and finally mixed with gelatin using 3 different proportions of the fibrinogen, alginate and gelatin solutions (compositions referred to as "FAG") or the Dermial, alginate and gelatin solutions (compositions referred to as "DAG"), see Table 1. Those bioinks were either manually molded to create cylindrical hydrogels, or ink jetted to create micrometric hydrogel beads (microbeads).

*Analysis of bioink viscosity.* Bioink formulations were analyzed to investigate their rheological properties at different temperatures and shear rates using a Discovery HR-2 (DHR2, TA Instruments). This allows the determination of the viscosity value of the hydrogels as a function of the experimental conditions to understand the behavior of the bioinks during flow and inkjet ejection. The measurement of the bioink viscosities with the DHR2 rheometer was done at shear rates between 0.01 - 100 s$^{-1}$ at temperatures of 25, 28, 30, 35 and 40C. *In silico* numerical simulation of shear stress experienced by cells within the hydrogel formulation was performed by Computational Fluid Dynamic (CFD) on ANSYS FLUENT® 2020 R1 Academic. ANSYS FLUENT® is a commercially available fluid dynamics (CFD) software which uses a Finite Volume Method (FVM) to perform CFD calculations.

*Inkjet bioprinting.* A PICO Pμlse® (Nordson EFD) jet valve was used as the inkjet device. Bioinks where stocked at 37C inside an incubator until use. Inkjet nozzles of 50 and 100 μm were used. Printing parameters used were: 10 ms aperture time, 4 bars of pressure, and printing temperatures between 28 - 37°C. The jetting system was kept at 37°C, positioned to jet directly into a cross-linking bath (recovery bath). The cross-linking bath was maintained inside an incubator (pre-warmed at 37°C) until jetting. During the jetting process (the time varies), for example 20 min, the cross-linking bath temperature decreased to room temperature in these experiments, although it would advantageously be maintained at 37°C. Once printed, the microbead lot is kept in the cross-linking bath in the incubator at 37°C for at least 20 min. They are then washed with NaCl 0.9% and resuspend in their culture media (see below).

*Cellularised structure consolidation.* See Fig. 3, step 340. A 30 min incubation at 37C in a solution of 3% CaCl$_2$ (Sigma), 0.4% transglutaminase (TAG; Activa WM, Ajinomoto, France), and 10U/ml Thrombin (filter-sterilized; Sigma; only when using FAG solutions) was used to cross-link the hydrogels. Then, three NaCl 0.9% washes of 5 min were used to wash away the calcium residues from the samples. Once the cross-linking ended, samples were placed in multi-well culture plates in culture MSC GROWTH culture media.

*Hydrogel dissolution.* Samples were first washed with PBS, and then incubated with 0.5 mL collagenase A (3%; Sigma) for 10 - 15 min at 37C. Two protocols were used (detailed below). Both protocols were found suitable but the second protocol including a single incubation with both the alginate lyase and the collagenase was found to cause less stress to the cells.

Protocol 1:

- Incubate 10 - 15 min using 0.5 mL of Collagenase at 37°C.
- Inactivate using 1 mL of culture media.
- Centrifuge at 1200 rpm for 5 min.
- Incubate 3 - 5 min using 0.5 mL of alginate lyase (A1603-100MG Sigma-Aldrich; 0.5mg/mL dissolved in 100 mM sodium citrate) at 37°C.
- Inactivate using 1 mL of culture media.
- Centrifuge at 1200 rpm for 5 min.
- Resuspend the cell pellet using 1 mL of culture media.

Protocol 2:

- Incubate 10- 18 min using 0.5 mL of Collagenase + 0.5 mL of alginate lyase at 37°C, with eventual manual agitation.
- Inactivate using 1 mL of culture media.
- Centrifuge at 1200 rpm for 5 min.
- Resuspend the cell pellet using 1 mL of culture media.

*Cell Viability Assay.* Cell viability in the samples was determined on different time points (such as after 1, 3, 5, 7, 14 and 21 days after production using the Live/Dead™ Viability/Cytotoxicity Kit (Invitrogen). The samples were incubated in PBS containing calcein AM (2 $\mu$M) and ethidium homodimer (4 $\mu$M) at 37C for 30 min to stain live (in green) and dead cells (in red).

*Quantification of secreted lactic acid.* Culture spent medium was collected in the 12-well plates before each media exchange. The lactic acid concentration was quantified using the L-Lactic Acid Assay from Megazyme (L-Lactic Acid (L-Lactate) Assay Kit, K-LATE, Megazyme) for auto-analyzer procedures. The assay was performed in 96-well plates. 200 $\mu$L of reactive R1 was deposited in the wells followed by addition of 10 $\mu$L of the samples and 25 $\mu$L of reactive R2. Each sample was deposited twice (duplicate) in the 96 well plate. The plates were incubated at 25C in the dark for 10 min before measurement of the absorbance at 340 nm with a spectrophotometer (TECAN infinite®). If the samples were too concentrated, they were diluted in deionized water. Fresh culture medium was used as a negative control for the supernatant samples.

*Growth monitoring by microcarrier measurement.* Microscopic pictures of ink jetted or manually molded hydrogel beads were taken. Using microscope scale bars, pictures were analyzed using the software ImageJ to measure the diameters of at least 6 to 10 samples (due to a high variability in sample diameters).

*Microscopy.* Samples were photographed after cross-linking using the Nikon Micro TS2 FL optical microscope. Sample dimensions were measured with the Nikon microscopy software. Fluorescent microscopy images were also taken after live/dead staining (Calcein/ethidium homodimer, Invitrogen) to assess their viability overtime. Bright field, green and red fluorescence images were taken for that purpose. ImageJ software was then used to merge the different channels to obtain images and further analyze cell viability.

## Example 1: Bioink compositions suitable for microcarrier culture systems

A bioink based on three components, fibrinogen, alginate, and gelatin (FAG, default components) or Dermial, alginate and gelatin (DAG) was used as a standard bioink formulation for creating hydrogel microbeads using inkjet bioprinting. The components of the bioink can be varied to enable customization of the size and composition of the microcarrier beads. The different formulations of bioink used are summarized in Table 1. The different formulations are diluted in DMEM (without calcium; Sigma), or MSC growth medium (PromoCell).

Bioink formulations such as those described in Table 1 are able to form microbead culture systems, wherein cells grow on the outside surface and embedded inside the hydrogel bead (as illustrated on Figure 5).

**Table 1:** Bio-ink Formulations (Dermial® (Bioiberica): Hyaluronic acid (60-75%); glycosaminoglycans (including dermatan and chondroitin sulfate) (≥ 10%); and collagen (≥ 5%)). All concentrations are in weight / volume.

| Bioink | Fibrinogen | Alginate | Gelatin | Dermial® | Hyaluronic acid |
|---|---|---|---|---|---|
| FAG 242 | 2 % | 2 % | 5 % | - | - |
| FAG 224 | 2 % | 1 % | 10 % | - | - |
| FAG 251 | 2 % | 2,5 % | 2,5 % | - | - |

(continued)

| Bioink | Fibrinogen | Alginate | Gelatin | Dermial® | Hyaluronic acid |
|---|---|---|---|---|---|
| DAG (1) 242 | - | 2 % | 5 % | 1 % | - |
| DAG (1) 224 | - | 1 % | 10% | 1 % | - |
| DAG (1) 251 | - | 2,5 % | 2,5 % | 1 % | - |
| DAG (2) 242 | - | 2 % | 5 % | 2 % | - |
| DAG (2) 224 | - | 1 % | 10% | 2% | - |
| DAG (2) 251 | - | 2,5 % | 2,5 % | 2% | - |
| HAG (1) 242 | - | 2% | 5% | - | 1% |
| HAG (1) 224 | - | 1 % | 10% | - | 1 % |
| HAG (1) 251 | - | 2,5 % | 2,5 % | - | 1 % |
| HAG (2) 242 | - | 2 % | 5 % | - | 2 % |
| HAG (2) 224 | - | 1 % | 10% | - | 2 % |
| HAG (2) 251 | - | 2,5 % | 2,5 % | - | 2 % |

**Example 2: Investigating the Properties of Different Bioink Formulations**

*2.1 Degradation of Hydrogel*

Cylindrical, manually molded hydrogels (in a 24 multi-well plate; average diameter: 14 mm) made of FAG 242, 224 and 251 were measured over time up to 21 days to observe if they undergo degradation. Macroscopic images were taken to measure the surface area of the hydrogels on days 1, 3, 5, 7, 10, 14, and 21. No degradation or size reduction could be observed, although a slight swelling could be observed (Figure 6).

To investigate whether AD-MSCs may release soluble molecules that could degrade the hydrogels, the size of hydrogels incubated in multi-well plates with AD-MSCs growing on 2D was measured. FAG 242 and FAG 251 hydrogel balls were manually cast by releasing droplets of bioink into the cross-linking bath. The hydrogel balls were then transferred to a multi-well plate, wherein either 1 or 3 droplets were incubated in the presence of AD-MSCs. Media was changed every 48h to maintain cell growth. The hydrogel droplets did not undergo any significant degradation in either of the two assayed conditions, maintaining their average size for at least 27 days (Figure 7).

*2.2 Analysis of the Viscosity of Different Bioink Formulations*

*Analysis of Bioink Viscosity*

The rheological properties of the hydrogel formulations were assessed to investigate their viscosity during ink-jetting. Table 2 shows the zero-rate viscosity for each of the different formulations, at a range of temperatures from 25 to 40°C. The printability of the bioink was measured qualitatively, by assessing which conditions allowed for jetting of the bioink, and which did not. Compositions were considered "printable" if they could be efficiently and reproducibly ink jetted. Compositions were considered "too viscous to print" if the bioink failed to be dispensed from the inkjet nozzle. Compositions were considered at "threshold viscosity" if jetting of the bioink was possible but not reproducibly. Only FAG 251, the formulation with the lowest gelatin content was printable at a temperature of 25C, FAG 242 and 224 required printing temperatures of at least 28 and 30C, respectively. The measured viscosities were used to parameterise a mathematical model to calculate the shear stress applied to the cells, as explained below and shown in Table 5.

**Table 2:** Zero-rate viscosity of FAG 242, 224, and 251 bioink formulations at different temperatures. * = too viscous to print, ** = threshold viscosity, other points = printable viscosity.

| Zero-rate viscosity (Pa.s) | Bioinks | | |
|---|---|---|---|
| Temperature (C) | 242 | 224 | 251 |
| 25 | 429.97* | 3202.31 * | 0.23** |

(continued)

| Zero-rate viscosity (Pa.s) | Bioinks | | |
|---|---|---|---|
| Temperature (C) | 242 | 224 | 251 |
| 28 | 3.22** | 291.32* | 0.15** |
| 30 | 0.54 | 4.90** | 0.12 |
| 35 | 0.63 | 0.50 | 0.09 |
| 37 | - | 0.46 | 0.08 |
| 40 | 0.15 | 0.11 | 0.06 |

Plotting the viscosity against the shear rate showed that FAG 242 and 224 demonstrate shear thinning behaviour at high shear rates, at temperatures below 28°C, when the solution is more viscous (Figure 8, top and middle panels). At temperatures above 28°C, all of the hydrogel solutions tested demonstrate Newtonian behaviour. For FAG 251, Newtonian behaviour is observed at all temperatures (Figure 8, bottom panel). While shear thinning is beneficial for reducing the shear stress experienced by cells during the printing process, Newtonian fluid dynamics are better suited to inkjet printing as they allow for an unchanged viscosity during the printing process. Table 3 shows the rate index for each formulation at each temperature, which distinguishes between shear thinning and Newtonian behaviour.

| Rate index | Bioinks | | |
|---|---|---|---|
| Temperature (C) | 242 | 224 | 251 |
| 25 | 0.18 | 0.16 | 0.86 |
| 28 | 0.45 | 0.19 | 0.81 |
| 30 | 0.99 | 0.35 | 0.99 |
| 35 | 0.99 | 0.99 | 0.98 |
| 37 | - | 1.00 | 0.99 |
| 40 | 1 | 1.00 | 0.99 |

**Table 3:** Rate index of FAG 242, 224, and 251 bioink formulations at different temperatures. Grey shading = shear thinning behaviour, no shading = Newtonian behaviour.

**Table 4:** K (consistency index) of FAG 242, 224, and 251 bioink formulations at different temperatures.

| K (consistency index) | Bioinks | | |
|---|---|---|---|
| Temperature (C) | 242 | 224 | 251 |
| 25 | 11.42 | 3.49 | 0.03 |
| 28 | 0.91 | 3.68 | 0.10 |
| 30 | 822.82 | 0.53 | 0.29 |
| 35 | 1.35 | 1.70 | 0.43 |
| 37 | - | 532.20 | 0.44 |
| 40 | NA | 91.72 | 4.98 |

*2.3 Analysis of Shear Stress*

The parameters obtained from the rheological measurements were used to calculate the minimum and maximum shear stress experienced for each of the formulations (Table 5). Equation 1 was used for formulations that showed shear thinning behaviour, and Equation 2 for formulations that showed Newtonian behaviour. Overall, the shear rate generated by the different tested formulations ranged between 57 - 7730 $s^{-1}$. Calculated from such shear rate, the resulting shear stress is between $9.71 \cdot 10^{-1}$ - $4.87 \cdot 10^3$ Pa, a range in which cells can maintain high viability.

Equation 1:

$$\tau_{rz} = -K \cdot |\dot{\gamma}|^n$$

Equation 2:

$$\tau = \eta \cdot \dot{\gamma}$$

| Shear stress (Pa) | | | | | | |
|---|---|---|---|---|---|---|
| Temperature (C) | Stress with minimum shear rate | | | Stress with maximum shear rate | | |
| | 242 | 224 | 251 | 242 | 224 | 251 |
| 25 | 23.64 | 6.66 | 0.97 | 57.22 | 14.62 | 66.21 |
| 28 | 5.61 | 7.93 | 2.64 | 51.14 | 20.17 | 141.07 |
| 30 | 30.78 | 2.18 | 6.84 | 4174.20 | 12.17 | 927.60 |
| 35 | 35.91 | 28.5 | 5.13 | 4869.90 | 3865.00 | 695.70 |
| 37 | - | 26.22 | 4.56 | - | 3555.80 | 618.40 |
| 40 | 5.7 | 6.27 | 3.42 | 773.00 | 850.30 | 463.80 |

**Table 5:** Minimum and maximum shear stress reached for FAG 242, 224, and 251 bioink formulations at different jetting temperatures. Grey shading = shear thinning behaviour, no shading = Newtonian behaviour.

Shear stress experienced by the bioink solutions along the flow path inside the inkjet printer was simulated *in silico*, using Computational Fluid Dynamics (CFD) software. Figure 9 shows the results using alginate only, modelled assuming a viscosity of 0.081 Pa.s, a fluid density of 1035 $kg/m^3$, and a pressure of 1 bar. Table 6 shows the maximum shear stress of pure alginate calculated using the simulation, at different inlet pressure. As expected, the results on Figure 9 show that the maximum shear stress occurs in the nozzle region, where the fluid experiences strong velocity changes as it is forced through the aperture, and a greater inlet pressure increases the maximum shear stress of the fluid. This was confirmed at higher pressures and using the compositions in Table 1.

**Table 6:** Maximum shear stress of pure alginate calculated by simulation.

| *Pressure (bar)* | Maximum shear stress (Pa) |
|---|---|
| 0.25 | 20.94 |
| 0.50 | 66.90 |
| 0.75 | 327.87 |
| 1.00 | 368.49 |

*2.4 Analysis of Bioink flow Rate*

The effect of temperature and pressure during ink jetting was measured, to investigate how these parameters affected the flow rate of the bioink. As expected, FAG 251, being the less viscous formulation, had an increased flow rate under equivalent conditions compared to FAG 242 (Tables 7 and 8). The flow rate of pure alginate was also measured as a comparator (Table 9). Formulations with higher fluidity, like FAG 251, may be advantageous, as a larger amount of microbeads can be produced.

**Table 7:** Determination of flow rate achieved for FAG 242, when jetting was performed under varied temperature and pressure.

| T (C) | P (bar) | Jetting time (s) | Mass jetted (g) | Volume jetted (μL) | Flow rate (μL/s) |
|---|---|---|---|---|---|
| 27 | 2 | | 0.017 | 16.634 | 1.109 |
| | 4 | 15 | 0.140 | 136.986 | 9.132 |
| 28 | 2 | | 0.056 | 54.795 | 3.653 |
| | 4 | | 0.144 | 140.900 | 9.393 |
| 29 | 2 | | 0.038 | 37.182 | 2.479 |
| | 4 | | 0.193 | 188.845 | 12.590 |
| 33 | 2 | | 0.079 | 77.299 | 5.153 |
| | 4 | | 0.294 | 287.671 | 19.178 |

**Table 8:** Determination of flow rate achieved for FAG 251, when jetting was performed under varied temperature and pressure.

| T (C) | P (bar) | Jetting time (s) | Mass jetted (g) | Volume jetted (μL) | Flow Rate (μL/s) |
|---|---|---|---|---|---|
| 26 | 0.25 | | 0.011 | 10.859 | 0.724 |
| | 0.5 | | 0.035 | 34.551 | 2.303 |
| | 0.75 | | 0.192 | 189.536 | 12.636 |
| | 1 | | 0.171 | 168.806 | 11.254 |
| 27 | 0.25 | 15 | 0.017 | 16.782 | 1.119 |
| | 0.5 | | 0.058 | 57.256 | 3.817 |
| | 0.75 | | 0.186 | 183.613 | 12.241 |
| | 1 | | 0.181 | 178.677 | 11.912 |
| 33 | 0.25 | | 0.065 | 64.166 | 4.278 |
| | 0.5 | | 0.128 | 126.357 | 8.424 |
| | 0.75 | 25 | 0.325 | 320.829 | 12.833 |
| | 1 | 15 | 0.28 | 276.407 | 18.427 |

**Table 9:** Determination of flow rate achieved for pure alginate, when jetting was performed under varied temperature and pressure.

| T (C) | P (bar) | Jetting time (s) | Mass jetted (g) | Volume jetted (μL) | Flow Rate (μL/s) |
|---|---|---|---|---|---|
| 26 | 0.25 | 15 | 0.047 | 46.813 | 3.121 |
| | 0.5 | 15 | 0.217 | 216.135 | 14.409 |
| | 0.75 | 15 | 0.374 | 372.510 | 24.834 |
| | 1 | 15 | 0.612 | 609.562 | 40.637 |

(continued)

| T (C) | P (bar) | Jetting time (s) | Mass jetted (g) | Volume jetted (μL) | Flow Rate (μL/s) |
|---|---|---|---|---|---|
| 27 | 0.25 | 15 | 0.123 | 122.510 | 8.167 |
| | 0.5 | 15 | 0.288 | 286.853 | 19.124 |
| | 0.75 | 15 | 0.045 | 44.821 | 2.988 |
| | 1 | 15 | 0.052 | 51.793 | 3.453 |
| 33 | 0.25 | 15 | 0.15 | 149.402 | 9.960 |
| | 0.5 | 15 | 0.419 | 417.331 | 27.822 |
| | 0.75 | 15 | 0.622 | 619.522 | 41.301 |
| | 1 | 15 | 0.52 | 517.928 | 34.529 |

## Example 3: Characterisation of Adipose-derived Mesenchymal Stem Cells (AD-MSCs)

When maintained in standard culture conditions AD-MSCs showed plastic-adherent growth, and expression over 99% of positive stemness surface markers (CD90, CD105, CD73, and CD44), with expression of negative stemness markers at less than 1% (Figure 10).

Differentiation of AD-MSCs cultured under normal conditions was also investigated, using a tripotentiality assay, to assess differentiation to adipogenic, osteogenic, and chondrogenic cell populations. Adipogenic-differentiated AD-MSCs were stained with Red oil to stain lipid droplets in the adipogenic cells, showing differentiation of a high proportion of the AD-MSCs (Figure 11, middle panel). Osteogenic-differentiated cells were stained with Alizarin red to stain calcium crystals in osteogenic cells, showing differentiation of a high proportion of the AD-MSCs (Figure 11, right panel).

Chondrogenic differentiation of AD-MSCs was assessed by quantifying glycosaminoglycan (GAG) production. Differentiated AD-MSCs had a significantly higher GAG content compared to control AD-MSCs (Figure 12).

Having characterised the cells in standard adherent cell culture conditions, these were then included in microcarriers according to the present disclosure.

## Example 4: Culturing AD-MSCs in Hydrogel Beads

FAG 242, 224, and 251 hydrogels loaded with AD-MSCs were manually molded into a cylindrical shape approximately 14 mm in diameter using 24-well multi-well plates and cultured for 21 days in 12-well multi-well plates at 37C in a 5% $CO_2$ atmosphere. Cell viability was measured on days 1, 3, 5, 7, 14, and 21. FAG 242 and 251 maintained cell viability above 80% throughout the 21 days of culture (Figure 13A and B).

Lactate levels were also measured to provide a read out of cell proliferation, by plotting the cumulative lactate concentration of the culture medium. Cells cultured in FAG 242 and FAG 251 hydrogel showed a faster increase in lactate production than FAG 224, indicating cells cultured in these conditions proliferate faster (Figure 13C).

FAG 242 and 251 were assayed to further investigate how their interaction would impact AD-MSCs bioproduction, and as a reference point for testing other bioink formulations. To enhance their biomimicry, DAG 242 and 251 formulations were also tested where Dermial® was used instead of the fibrinogen (although they can also be used in combination), as it helps to replicate biological molecules that are found in the human native extracellular matrix. To do this, the bioinks were cast manually as a first step, and then ink jetted to create microbeads using either a 100μm nozzle or a 50μm nozzle.

Cell numbers in the hydrogel microbeads were then counted on days 2 and 12 of culture. This showed that all four bioink formulations could support cell proliferation, with around a 3- to 4-fold increase in cell numbers in each of the different formulations (Figure 14B and C).

Stemness markers were also assessed on days 2 and 21 of culture with flow cytometry, to ensure culture in the hydrogel microbeads did not induce differentiation. Results for FAG 251 day 2 were lost, however for all formulations a high proportion of cells maintained expression of stemness markers during at least the 12 days post-encapsulation (Figure 14D), as measured by flow cytometry.

## Example 5: Inkjet Bioprinting with 100 um Nozzle

FAG 242 and 251, and DAG 242 and 251 bioinks were produced manually, and ink jetted to create microbeads using a 100 μm nozzle (Figure 15A). Using the 100 μm nozzle generated microbeads sized between 400 - 1000 μm

during the first 14 days of culture (Figure 15B).

Cell viability was assessed at days 2, 8, 14 and 21 (Figures 15E and F). The DAG bioink formulations were found to provide superior cell viability compared to the FAG formulations (Figures 15C and D), however, this was likely due to protocol optimisation. It was found that the temperature of the bioink impacted its printability in the inkjet device, which was optimised to stabilize it at 37°C by using a syringe heater. Maintaining an optimal temperature during the printing process prevents a change in the viscosity of the bioink, which stabilises cell viability.

In some cases, microbeads aggregated and grew in size at day 21 (in the static culture conditions investigated - this is not expected to be the case in an agitated vessel), reaching sizes around the 2 mm (Figure 15B). The H&E staining in Figure 15G shows that this is due to the cells producing an extracellular matrix that wraps around the hydrogel microbeads. These images also demonstrate that cells grow on the surface of the microbeads and also embedded inside of them.

### Example 6: Inkjet Bioprinting with 50 um Nozzle

AD-MSC-loaded microbeads were produced by ink-jetting the two FAG formulations through a 50 $\mu$m nozzle (Figure 16A). Using this nozzle the microbeads produced ranged between 100 - 400 $\mu$m in size over 21 days in culture (Figure 16B).

Cell viability in microbeads made from the FAG formulations was high throughout the 21 days culture (Figure 16C). Cell viability was higher when the syringe used during the printing process was kept warm (as was the case in the results of Figure 16C, but not in the results of Figure 15C). The results of Figure 16C show that with the 100$\mu$m nozzle, extremely high viability is expected by keeping the bioink composition at 37°C until printing (since the larger nozzle should be even more favourable to cell viability). On days 2 and 21 the microbeads were dissolved and the cells counted, showing that the FAG bioink formulations ink jetted using the 50 $\mu$m nozzle were able to support 3- to 6-fold expansion of AD-MSCs (Figures 16D, E, and F).

The presence of cell surface stemness markers was also verified, showing that most stem cell markers remained high and negative markers of stemness remained low **(Figure 16G).** Further improvements of these are expected by optimisation of the cell dissociation protocol. Nevertheless, the data shows that the methods described are compatible with maintenance of pluripotent cells.

### *Equivalents and Scope*

All documents mentioned in this specification are incorporated herein by reference in their entirety.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

**Claims**

1.  A method of producing a microcarrier for cell culture, the method comprising:

    providing a solution comprising one or more biomaterials capable of forming a hydrogel and one or more cells; and
    propelling a droplet of the solution into a liquid medium by ink jetting, thereby producing a cellularised microcarrier.

2.  The method of claim 1, wherein the one or more cells comprise or consist of one or more stem cells and/or wherein the cells are present at a concentration of at least 0.5 million cells / mL, at least 1 million cells / mL, or between 1 and 3 million cells /mL.

3.  The method of any preceding claim, wherein the solution further comprises a liquid culture medium and/or wherein the solution has been obtained by mixing one or more solutions each comprising biomaterials capable of forming a hydrogel and/or cells in a liquid culture medium, and/or wherein the solution further comprises a compound or composition that promotes cell adhesion, optionally wherein the compound or composition is selected from fibrinogen, Dermial®, hyaluronic acid, chitosan, collagen I, and combinations thereof.

4.  The method of any preceding claim, wherein the droplet has a diameter between $100\mu m$ and 2mm, between $100\mu m$ and 1.5mm, between $100\mu m$ and 1mm, optionally between $100\mu m$ and $800\mu m$, and/or wherein the droplets comprise a volume of solution below 5 $\mu l$, below 1.7 $\mu l$, below 0.5 $\mu l$, or between 0.5 nl and 0.3 $\mu l$.

5.  The method of any preceding claim, wherein the method further comprises consolidating the droplet, optionally wherein consolidating the droplet comprises exposing the droplet to one or more solutions that cross-link one or more of the biomaterials capable of forming a hydrogel and/or wherein consolidating produces a microstructure comprising a hydrogel matrix and the one or more cells, and/or wherein the liquid medium in which the droplet is propelled comprises one or more compounds capable of cross-linking one or more of the biomaterials capable of forming a hydrogel.

6.  The method of any one of the preceding claims, wherein the step of propelling a droplet of the composition is performed:

    (i) at a temperature between 20 and 40°C, or between 30 and 40°C;
    (ii) using an inkjet printer with a fluid pressure between 0.25 and 5 bar, between 2 and 5 bar, optionally between 3 and 4 bar; and/or
    (iii) using an ink jetting device with a valve opening time between 5 and 50ms, optionally about 10 ms, and/or wherein the droplet is propelled using a flow rate between about $9\mu L/s$ and about $20\mu L/s$, or between 15 $\mu L/s$ and $20\mu L/s$.

7.  The method of any preceding claim, wherein the biomaterial capable of forming a hydrogel comprises alginate and/or gelatin, optionally wherein the solution comprises at most 5% w/v gelatin or at most 3% w/v of gelatin, and/or wherein the solution comprises alginate and gelatin, optionally wherein the solution comprises between 2% and 10% w/v of gelatin, and between 0.5% and 3% w/v of alginate, and/or wherein the solution comprises about 5% w/v gelatin and about 2%w/v alginate, about 2.5% w/v gelatin and about 2% w/v alginate.

8.  The method of any preceding claim, wherein the viscosity of the solution comprising one or more biomaterials capable of forming a hydrogel and one or more cells is below 5 Pa.s, below 1 Pa.s or between 0.05 and 0.7 Pa.s.

9.  The method of any preceding claim, wherein the method comprises collecting the droplet in a non-static liquid medium, optionally wherein the liquid medium is in an agitated vessel such as a bioreactor, and/or wherein the liquid medium into which the droplet is propelled is agitated, optionally with an impeller.

10. The method of any preceding claim, wherein:

the solution comprises about 2% w/v fibrinogen, about 2.5% w/v alginate, and about 2.5% w/v gelatin, and wherein the step of propelling a droplet of the composition is performed at a temperature between 30°C and 40°C and/or in conditions such that the viscosity of the solution is between 0.06 - 0.12 Pa.s; or
the solution comprises about 2 % w/v fibrinogen, about 2% w/v alginate, and about 5% w/v gelatin, and wherein the step of propelling a droplet of the composition is performed at a temperature between 30°C and 40°C and/or in conditions such that the viscosity of the solution is between 0.15 and 0.63 Pa.s; or
the solution comprises about 2% w/v fibrinogen, about 1% w/v alginate, and about 10% w/v gelatin, and wherein the step of propelling a droplet of the composition is performed at a temperature between 35°C and 40°C and/or in conditions such that the viscosity of the solution is between 0.11 - 0.63 Pa.s.

11. The method of any preceding claim, wherein propelling a droplet of the composition is performed using a continuous ink jetting device, and/or wherein propelling a droplet of the composition is performed using a device comprising a piezoelectric valve, and/or wherein propelling a droplet of the composition is performed in a sterile environment, and/or wherein propelling a droplet of the composition is performed using an apparatus configured such that every element of the apparatus flow path is sterilisable.

12. The method of any preceding claim, wherein propelling a droplet comprises applying pressure on the solution passing through a nozzle, optionally wherein the nozzle has a diameter between about $50\mu$m and about 300 $\mu$m, between about $50\mu$m and about 200 $\mu$m, or between about $50\mu$m and about 100 $\mu$m and/or wherein the nozzle has a diameter of about 50 $\mu$m and the droplet has a diameter between 100 $\mu$m and 400 $\mu$m, or wherein the nozzle has a diameter of about 100 $\mu$m and the droplet has a diameter between 400 $\mu$m and 1000 $\mu$m.

13. A cellularised microcarrier obtained or obtainable through the method of any preceding claim.

14. A method for performing a bioproduction process comprising the production of a cell population, wherein the process comprises maintaining a microcarrier according to claim 18 in suspension in a culture medium in a culture vessel, optionally wherein the culture vessel is an agitated bioreactor and/or wherein the method further comprises culturing the cells for at least 3, 5, 7, 14, 21, or 28 days, or about 14, days, 21 days, 28 days, 35 days, 42 days, 49 days, 60 days, about 1 month or about 2 months, and/or maintaining the cells for at least 4, at least 10 or at least 20 amplifications, and/or wherein maintaining the cells comprises maintaining the cells at least partially embedded in the microcarrier in suspension in the culture medium without adding additional physical support for cell growth, and/or wherein the cells are stem cells and wherein culturing the cells comprises maintaining the cells in an undifferentiated state.

15. A system comprising:

a bioreactor, optionally wherein the bioreactor is a stirred tank;
an inkjet printing device coupled with the bioreactor or with a microcarrier recovery tank fluidically connected with the bioreactor, wherein the inkjet printing device, bioreactor and optionally microcarrier recovery system are all connected in a single sterile system and wherein the inkjet printing device is configured to dispense droplets of liquid directly into a liquid medium in the bioreactor or in the microcarrier recovery tank.

120

PROVIDE BIO-INK COMPOSITION

FORM DROPLETS OF BIO-INK ~130

CONSOLIDATE BIO-INK MICROBEADS ~140

150

CULTURE CELL POPULATION IN 3D HYDROGEL MICROBEADS

HARVEST CELLULAR PRODUCT ~160

Fig. 1A

Fig. 1B

Fig. 2

320

Fibrinogen  Alginate  Gelatin  Cells

CULTIVATION IN INCUBATOR OR BIOREACTOR

330  ~  10 min incubation at 37 °C

360

3D BIOPRINTING  HYDROGEL RETICULATION

340  ~

350

Fig. 3

Fig. 4

Fig. 5

Fig. 6

Fig. 7

242

224

251

Fig. 8

Fig. 9

Fig. 10

CD44 (+)

Negative markers: CD45, CD34, CD11b, CD19, and HLA-DR

Fig. 10 (continued)

Fig. 11

Fig. 12

Fig. 13

B

Fig. 13 (continued)

Fig. 14

Fig. 14D

Fig. 15

Fig.15C

Fig. 15D

Fig. 15 (Continued)

Fig. 15G

A

FAG 242

FAG 251

C

FAG 242    FAG 251

Fig. 16

Fig. 16 (Continued)

Fig. 16 (continued)

Europäisches
Patentamt

European
Patent Office

Office européen
des brevets

# EUROPEAN SEARCH REPORT

Application Number

EP 23 30 5244

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/189723 A1 (FELDER ROBIN A [US] ET AL) 25 July 2013 (2013-07-25) * paragraphs [0024], [0034], [0035], [0037]; figure 4 * ----- | 1-15 | INV. C12M1/12 C12M1/26 C12N5/00 C12N11/04 B29C64/112 |
| X,D | US 11 511 254 B2 (UNIV BORDEAUX [FR]; CENTRE NAT RECH SCIENT [FR] ET AL.) 29 November 2022 (2022-11-29) * column 3, line 15 - column 4, line 67 * * column 6, lines 43-46,57-67; example 1 * ----- | 1,3-5,7, 11,13,14 | |
| X | KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 1 January 2016 (2016-01-01), pages 1593-1604, XP055393107, UK ISSN: 1473-0197, DOI: 10.1039/C6LC00133E * figure 3 * ----- -/-- | 1,13,14 | |

**TECHNICAL FIELDS SEARCHED (IPC)**

C12M
C12N
C12R
B29C

The present search report has been drawn up for all claims

| Place of search | Date of completion of the search | Examiner |
|---|---|---|
| Berlin | 19 July 2023 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 23 30 5244

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IKKI HORIGUCHI ET AL: "Alginate Encapsulation of Pluripotent Stem Cells Using a Co-axial Nozzle", JOURNAL OF VISUALIZED EXPERIMENTS, JOVE, US , vol. 101 2 July 2015 (2015-07-02), pages 1-7 e52835, XP009523467, ISSN: 1940-087X, DOI: 10.3791/52835 Retrieved from the Internet: URL:http://www.jove.com/video/52835 * 1: Preparing Materials; 2: Cell Encapsulation Into Alginate Hydrogel Capsules; pages 1,2 * | 1-3,5,7, 9,11-14 | |
| X | US 5 264 359 A (ENAMI JUNPEI [JP] ET AL) 23 November 1993 (1993-11-23) * column 2, line 45 - column 3, line 26; example 1 * * column 4, line 36 - column 6, line 17; figures 1,2 * | 1,3,5,7, 9,11-14 | |

TECHNICAL FIELDS SEARCHED (IPC)

The present search report has been drawn up for all claims

| Place of search | Date of completion of the search | Examiner |
|---|---|---|
| Berlin | 19 July 2023 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5244

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2023

| Patent document cited in search report | | | Publication date | Patent family member(s) | | | Publication date |
|---|---|---|---|---|---|---|---|
| US 2013189723 | A1 | | 25-07-2013 | AU | 2004260106 | A1 | 03-02-2005 |
| | | | | CA | 2532754 | A1 | 03-02-2005 |
| | | | | CN | 101416059 | A | 22-04-2009 |
| | | | | EP | 1660629 | A2 | 31-05-2006 |
| | | | | IL | 173103 | A | 30-01-2014 |
| | | | | JP | 2007535902 | A | 13-12-2007 |
| | | | | US | 2005054101 | A1 | 10-03-2005 |
| | | | | US | 2012009559 | A1 | 12-01-2012 |
| | | | | US | 2013189723 | A1 | 25-07-2013 |
| | | | | WO | 2005010162 | A2 | 03-02-2005 |
| US 11511254 | B2 | | 29-11-2022 | BR | 112020010141 | A2 | 10-11-2020 |
| | | | | CN | 111801156 | A | 20-10-2020 |
| | | | | DK | 3713665 | T3 | 01-11-2021 |
| | | | | EP | 3713665 | A1 | 30-09-2020 |
| | | | | ES | 2895935 | T3 | 23-02-2022 |
| | | | | FR | 3073751 | A1 | 24-05-2019 |
| | | | | IL | 274789 | A | 30-07-2020 |
| | | | | JP | 7082358 | B2 | 08-06-2022 |
| | | | | JP | 2021504132 | A | 15-02-2021 |
| | | | | KR | 20200090200 | A | 28-07-2020 |
| | | | | RU | 2020120192 | A | 23-12-2021 |
| | | | | SG | 11202004780T | A | 29-06-2020 |
| | | | | US | 2020360888 | A1 | 19-11-2020 |
| | | | | WO | 2019101734 | A1 | 31-05-2019 |
| US 5264359 | A | | 23-11-1993 | DE | 68913890 | T2 | 04-08-1994 |
| | | | | EP | 0364606 | A1 | 25-04-1990 |
| | | | | US | 5264359 | A | 23-11-1993 |
| | | | | WO | 8910397 | A1 | 02-11-1989 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20190002836 A
- EP 2022073485 W
- WO 2008075206 A2
- US 11511254 B2
- US 2022235320 A1

### Non-patent literature cited in the description

- **POURCHET et al.** *Adv. Healthcare Mater.,* 2017, 1601101
- **WONG, H. L. et al.** A 3D collagen microsphere culture system for GDNF-secreting HEK293 cells with enhanced protein productivity. *Biomaterials,* 2007, vol. 28 (35), 5369-5380
- **ZWEIGERDT, R.** Large Scale Production of Stem Cells. *Advances in Biochemical Engineering/Biotechnology,* 2009, 1-35
- **SILVA COUTO, P. et al.** Expansion of human mesenchymal stem/stromal cells (hMSCs) in bioreactors using microcarriers: lessons learnt and what the future holds. *Biotechnology Advances,* 2020, vol. 45, 107636
- Microcarrier Culture Systems for Stem Cell Manufacturing. **BADENES, S. M. et al.** Stem Cell Manufacturing. Elsevier B.V, 2016